# EUROPEAN PATENT APPLICATION

(11) **EP 4 079 813 A1**
(43) Date of publication of application: **26.10.2022**
(21) Application number: 21170261.8
(22) Date of filing: 23.04.2021
(51) Int. Cl.: C09D 5/00

(54) **BUFFER COMPOSITION COMPRISING A FIRST AND A SECOND BUFFER COMPONENT**

(71) Applicant: Omya International AG, 4665 Oftringen (CH)
(72) Inventor: SCHOELKOPF, Joachim, 5727 Oberkulm (CH); GLAUBITZ, Joachim, 6264 Pfaffnau (CH); HETTMANN, Kai Max, 79639 Grenzach-Wyhlen (DE); SÜTTERLIN, Klaus, 79426 Buggingen (DE)
(74) Representative: Glas, Holger

(57) **Abstract**

The present invention relates to a buffer composition comprising a first and a second buffer component, an article comprising the buffer composition being an aqueous preparation and/or a solid article, a process for buffering an aqueous preparation and a solid article as well as the use of said buffer composition for maintaining the pH of article, preferably an aqueous preparation or a solid article, equal to or below 12.

## Description

The present invention relates to a buffer composition comprising a first and a second buffer component, an article comprising the buffer composition being an aqueous preparation and/or a solid article, a process for buffering an aqueous preparation and a solid article as well as the use of said buffer composition for maintaining the pH of an article, preferably an aqueous preparation or a solid article, equal to or below 12.

In practice, aqueous preparations and especially suspensions, emulsions, dispersions or slurries of water-insoluble solids such as minerals, fillers or pigments are used extensively in the paper, paint, rubber, and plastics industries as coatings, fillers, extenders and pigments for papermaking as well as aqueous lacquers and paints. For example, suspensions or slurries of calcium carbonate, talc or kaolin are used in the paper industry in large amounts as filler and/or as a component in the preparation of coated paper. Furthermore, such aqueous preparations are also used as additives in the concrete and agriculture industries. Typical aqueous preparations of water-insoluble solids are characterized in that they comprise water, a water-insoluble solid compound and optionally further additives, such as dispersing agents, in the form of a suspension, a slurry or dispersion with a water-insoluble solid content of 0.1 to 99.0 wt.-% based on the total weight of the preparation. A typical aqueous preparation is a White Mineral Dispersion (WMD) having a solids content of 45.0 to 78.0 wt.-%. Water-soluble polymers and copolymers which may be used as e.g. dispersant and/or grinding aid in such preparation are, for example, described in US5278248.

The aforementioned aqueous preparations are often subject to pH changes during storing and handling resulting in destabilization of the preparation such that the mechanical and optical properties can be affected. Therefore, the manufacturers of such aqueous preparations usually take measures for stabilising the suspensions, dispersions or slurries pH by adding buffering components or corresponding compositions.

For example, pH stabilizing systems based on amines which are suitable for maintaining the pH of aqueous preparations are well known and widely used. However, the issue with such amines is that they may render the aqueous preparations very alkaline, i.e. above pH 12, which then necessitates specific work safety measures and specific labelling of the final products to be caustic. Furthermore, amines over time may undergo decomposition which may lead to a deterioration of the optical properties such as yellowing of the product and unpleasant smell.

Accordingly, it is an object of the present invention to provide a pH stabilizing system for maintaining the pH of articles such as aqueous preparations and solid articles. A further object of the present invention is to provide a pH stabilizing system without using amines. Another object of the present invention is to provide a pH stabilizing system keeping the aqueous preparation in a pH range below 12. A still further object of the present invention is to provide a pH stabilizing system which does not deteriorate the optical properties of the articles and does not adversely affect the smell of the article. Another object of the present invention is to provide a process for buffering articles accordingly.

The foregoing objects and other objects are solved by the subject-matter as defined herein in the independent claims.

According to one aspect of the present invention, a buffer composition comprising a first and a second buffer component is provided, wherein
a) the first buffer component is at least one water soluble or water dispersable source of magnesium ions and/or zinc ions, and
b) the second buffer component is at least one alkali carbonate and/or at least one alkali bicarbonate,
wherein the molar ratio of the first buffer component to the second buffer component [Mg and/or Zn ions/ carbonate and/or bicarbonate ions] is from 10 000:1 to 1:10 000.

According to another aspect of the present invention, an article being an aqueous preparation, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation, comprising the buffer composition as defined herein, and/or a solid article, preferably a coating, paint film, lacquer or coating, paper coating, paper, paperboard, adhesive, sealant, pigment, fiber, plaster, plaster-spray, plasterboard, binder, thickener and/or concrete, comprising the buffer composition as defined herein, is provided.

According to a further aspect of the present invention, a process for buffering an aqueous preparation is provided, said process comprising the steps of
a) providing an aqueous preparation, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation,
b) providing a buffer composition as defined herein, and
c) contacting and mixing the aqueous preparation of step a) one or more times with the buffer composition of step b) for obtaining the buffered aqueous preparation.

According to a still further aspect of the present invention, a process for buffering a solid article is provided, said process comprising the steps of
a) providing a solid article, preferably a coating, paint film, lacquer or coating, paper coating, paper, paperboard, adhesive, sealant, pigment, fiber, plaster, plaster-spray, plasterboard, binder, thickener and/or concrete comprising the buffer composition defined herein, and
b) moisten the surface of the solid article of step a) for obtaining the buffered solid article.

According to still another aspect of the present invention, the use of a buffer composition as defined herein for maintaining the pH of an article, preferably an aqueous preparation or a solid article, equal to or below 12, preferably from 4 to 12, more preferably from 6 to 11.5 and most preferably from 8.5 to 10.5, is provided.

The inventors surprisingly found out that the foregoing buffer composition provides a pH stabilization for aqueous preparations as well as solid articles, which are contacted with moisture or water. Furthermore, the buffer composition provides a pH stabilizing system without using amines and keeps the aqueous preparation in a pH range below 12. In addition thereto, the buffer composition does not deteriorate the optical properties of the preparation and does not adversely affect the smell of the preparation.

More precisely, the inventors found out that a pH stabilizing for articles, such as aqueous preparation as well as solid articles, can be achieved if a water soluble or water dispersable source of magnesium ions and/or zinc ions in combination with at least one alkali carbonate and/or at least one alkali bicarbonate is used in a specific molar ratio.

Advantageous embodiments of the inventive buffer composition, the processes and the use are defined in the corresponding sub-claims.

According to one embodiment, the at least one alkali carbonate is selected from the group consisting of sodium carbonate, potassium carbonate, lithium carbonate, and mixtures thereof, preferably sodium carbonate and/or lithium carbonate and most preferably lithium carbonate and/or the at least one alkali bicarbonate is selected from the group consisting of sodium bicarbonate, potassium bicarbonate, lithium bicarbonate, and mixtures thereof, preferably sodium bicarbonate and/or lithium bicarbonate and most preferably lithium bicarbonate.

According to another embodiment, the at least one water soluble or water dispersable source of magnesium ions is at least one magnesium ion-comprising material, preferably the at least one magnesium ion-comprising material is selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, preferably magnesium oxide and/or magnesium hydroxide; and/or the at least one water soluble or water dispersable source of zinc ions is at least one zinc ion-comprising material, preferably the at least one zinc ion-comprising material is selected from the group comprising zinc carbonate, zinc oxide, zinc hydroxide, zinc phosphate and mixtures thereof, preferably zinc oxide.

According to another embodiment, the molar ratio of the first buffer component to the second buffer component [Mg and/or Zn ions / carbonate and/or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

According to yet another embodiment, the buffer composition is capable of maintaining the pH of an article, preferably an aqueous preparation or a solid article, equal to or below 12, preferably from 4 to 12, more preferably from 6 to 11.5 and most preferably from 8.5 to 10.5.

According to one embodiment, the buffer composition is in form of a powder, granules, pellets or tablets.

According to another embodiment, the buffer composition comprises a solvent, preferably an organic solvent and/or water.

According to yet another embodiment, the buffer composition is in form of a solution, dispersion, suspension or paste, preferably an aqueous solution, dispersion, suspension or paste.

According to another embodiment, the buffer composition is free of buffer components comprising ammonia, methylamine, dimethylamine, trimethylamine, diethylamine, propylamine, butylamine, hexylamine, amino-2-methylpropanol (AMP), monoethanolamine (MEA), monoisopropanolamine (MIPA), triethylenetetramine (TETA), calcium hydroxide and mixtures thereof, preferably amine-based buffer components, monoalcohol-based buffer components, primary alkanol amine-based buffer components, hydroxide-based buffer components and mixtures thereof.

According to one embodiment of the article, the article further comprises
(i) at least one inorganic particulate material, preferably the at least one inorganic particulate material is selected from the group comprising natural ground calcium carbonate, natural and/or synthetic precipitated calcium carbonate, surface-reacted calcium carbonate, dolomite, calcium sulphate, kaolin, clay, barite, talcum, quartz, mica, gypsum, aluminium hydroxide, aluminium silicate, titanium dioxide, magnesite, hydromagnesite, hydroxylapatite, perlite, sepiolite, brucite and mixtures thereof, and most preferably the at least one inorganic particulate material comprises natural ground calcium carbonate and/or synthetic precipitated calcium carbonate, and/or
(ii) at least one organic material, preferably the at least one organic material is selected from the group comprising carbohydrates such as starch, sugar, cellulose, modified cellulose and cellulose based pulp, glycerol, hydrocarbons, water-soluble polymers such as polyacrylates, and mixtures thereof.

According to another embodiment of the article, the article is an aqueous preparation having
(i) a pH value of equal to or below 12, preferably from 4 to 12, more preferably from 6 to 11.5 and most preferably from 8.5 to 10.5, and/or
(ii) a solids content of up to 85.0 wt.-%, preferably from 10.0 to 82.0 wt.-%, and more preferably from 20.0 to 80.0 wt.-%, based on the total weight of the aqueous preparation.

According to yet another embodiment of the article, the article is an aqueous preparation and the first buffer component is present in a supersaturated state in the aqueous preparation and/or the second buffer component is present in a supersaturated state in the aqueous preparation.

According to one embodiment, the first and the second buffer components are present in the article in a total amount of at least 100 ppm, e.g. from 100 to 27 000 ppm, preferably at least 250 ppm, e.g. from 250 to 25 000 ppm, more preferably at least 500 ppm, e.g. from 500 to 20 000 ppm, still more preferably at least 600 ppm, e.g. from 600 to 15 000 ppm, even more preferably at least 750 ppm, e.g. from 750 to 10 000 ppm, and most preferably from 750 to 5 000 ppm, calculated relative to the total weight of the article.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

In the following, preferred embodiments of the inventive buffer composition will be set out in more detail. It is to be understood that these embodiments and details also apply to the inventive articles processes and use as far as applicable. Those skilled in the art will understand that many embodiments described herein can be combined or applied together.

### The buffer composition

According to the present invention, a buffer composition comprising a first and a second buffer component is provided. The first buffer component is at least one water soluble or water dispersable source of magnesium ions and/or zinc ions, and the second buffer component is at least one alkali carbonate and/or at least one alkali bicarbonate, wherein the molar ratio of the first buffer component to the second buffer component [Mg and/or Zn ions/carbonate and/or bicarbonate ions] is from 10 000:1 to 1:10 000.

Thus, it is one requirement of the present invention that the buffer composition comprises at least one water soluble or water dispersable source of magnesium ions and/or zinc ions as a first buffer component, and at least one alkali carbonate and/or at least one alkali bicarbonate as a second buffer component.

In the meaning of the present invention, the term "buffer composition" refers to a composition which keeps the pH of an article, preferably an aqueous preparation or a solid article that is moisten, in a certain pH range and thus stabilizes the pH.

According to the present invention, the wording "keeps the pH in a certain pH range" or "pH stabilizing" or "maintaining the pH" means that no significant pH change is observed in the article, e.g. the aqueous preparation or the solid article that is moisten, when the buffer composition is present. In particular, this preferably does not lead to an increase or decrease of the pH in the article, e.g. the aqueous preparation or the solid article that is moisten, compared to the article immediately after treatment, especially not to an increase or decrease of the pH value to more than 2, preferably to more than 1.

It is one requirement of the present invention that the buffer composition comprises at least one water soluble or water dispersable source of magnesium ions and/or zinc ions as a first buffer component.

The term source of "at least one" water soluble or water dispersable source of magnesium ions and/or zinc ions in the meaning of the present invention means that the source comprises, preferably consists of, one or more water soluble or water dispersable source(s) of magnesium ions and/or zinc ions.

In one embodiment of the present invention, the water soluble or water dispersable source of magnesium ions and/or zinc ions comprises, preferably consists of, one water soluble or water dispersable source of magnesium ions and/or zinc ions. Alternatively, the water soluble or water dispersable source of magnesium ions and/or zinc ions comprises, preferably consists of, two or more water soluble or water dispersable sources of magnesium ions and/or zinc ions. For example, the water soluble or water dispersable source of magnesium ions and/or zinc ions comprises, preferably consists of, two or three water soluble or water dispersable sources of magnesium ions and/or zinc ions. Preferably, the water soluble or water dispersable source of magnesium ions and/or zinc ions comprises, preferably consists of, two or more water soluble or water dispersable sources of magnesium ions and/or zinc ions

It is appreciated that the at least one water soluble or water dispersable source of magnesium ions and/or zinc ions of the instant buffer composition can be any material comprising, preferably consisting of, magnesium ions and/or zinc ions as ions.

It is appreciated that the at least one source of magnesium ions and/or zinc ions is water soluble or water dispersable.

Accordingly, the term "water soluble" or "soluble in water" in the meaning of the present invention refers to systems in which the source of magnesium ions and/or zinc ions forms a solution with water, i.e. the particles of the at least one source of magnesium ions and/or zinc ions are dissolved in the solvent. Alternatively, the term "water dispersable" or "dispersable in water" in the meaning of the present invention refers to systems in which only a part of the source of magnesium ions and/or zinc ions forms a solution with water, i.e. only a part of the particles of the at least one source of magnesium ions and/or zinc ions are dissolved in the solvent.

The term "source" of magnesium ions and/or zinc ions in the meaning of the present invention refers to a compound that comprises, preferably consists of, magnesium ions and/or zinc ions, i.e. magnesium ions and/or zinc ions.

It is to be noted that the at least one source of magnesium ions and/or zinc ions can be a source comprising magnesium ions and zinc ions. Alternatively, the at least one source of magnesium ions and/or zinc is a source comprising magnesium ions or zinc ions.

Preferably, the at least one source of magnesium ions and/or zinc is a source comprising magnesium ions or zinc ions as ions. More preferably, the at least one source of magnesium ions and/or zinc ions is a source comprising zinc ions as ions.

In one embodiment, the ions of the at least one source of magnesium ions and/or zinc ions consists of magnesium ions and/or zinc ions. For example, the ions of the at least one source of magnesium ions and/or zinc ions consists of magnesium ions and zinc ions. Alternatively, the ions of the at least one source of magnesium ions and/or zinc ions consists of magnesium ions or zinc ions. Preferably, the ions of the at least one source of magnesium ions and/or zinc ions consists of zinc ions.

In one embodiment of the present invention, the at least one water soluble or water dispersable source of magnesium ions is at least one magnesium ion-comprising material. Preferably the ionic group of the at least one magnesium ion-comprising material is selected from the group comprising oxide, hydroxide, phosphate, sulphate, chloride, bromide, and mixtures thereof. In particular, the at least one magnesium ion-comprising material is selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof.

For example, the at least one water soluble or water dispersable source of magnesium ions is preferably magnesium oxide and/or magnesium hydroxide. Preferably, the at least one water soluble or water dispersable source of magnesium ions is magnesium oxide.

The at least one water soluble or water dispersable source of magnesium ions may be also present as a polymeric salt of magnesium, such as acrylic homopolymers, acrylic copolymers such as copolymers of acrylic acid and maleic acid and/or acrylamide, polyphosphates and mixtures thereof having multiple acidic sites which can be partially or totally neutralised with magnesium ions. The polymeric salt of magnesium is preferably magnesium polyacrylate.

The polymeric salt of magnesium is preferably partially or completely neutralized, preferably to a degree of 5.0 to 100.0 %, preferably to a degree of 25.0 to 100.0 % and most preferably to a degree of 75.0 to 100.0 % using a neutralizing agent containing ions of magnesium and, optionally other alkali metals and/or alkaline earth metals. In one embodiment, the acidic sites of the polymeric salt of magnesium are neutralized using a neutralizing agent containing only magnesium. Neutralized polyacrylates and/or polymethacrylates with an average molecular weight of not more than 50 000, preferably with an average molecular weight in the range from 1 000 to 25 000 and more preferably in the range from 3 000 to 12 000 are especially suitable.

Additionally or alternatively, the at least one water soluble or water dispersable source of zinc ions is preferably provided in the form of at least one zinc ion-comprising material. Preferably the anionic group of the at least one zinc ion-comprising material is selected from the group comprising carbonate, oxide, hydroxide, phosphate, and mixtures thereof. In particular, the at least one zinc ion-comprising material is selected from the group comprising zinc carbonate, zinc oxide, zinc hydroxide, zinc phosphate, and mixtures thereof.

For example, the at least one water soluble or water dispersable source of zinc ions is preferably zinc carbonate, zinc oxide or zinc hydroxide. Preferably, the at least one water soluble or water dispersable source of zinc ions is zinc oxide.

The at least one water soluble or water dispersable source of zinc ions may be also present as a polymeric salt of zinc, such as acrylic homopolymers, acrylic copolymers such as copolymers of acrylic acid and maleic acid and/or acrylamide, polyphosphates and mixtures thereof having multiple acidic sites which can be partially or totally neutralised with zinc ions. The polymeric salt of zinc is preferably zinc polyacrylate.

The polymeric salt of zinc is preferably partially or completely neutralized, preferably to a degree of 5.0 to 100.0 %, preferably to a degree of 25.0 to 100.0 % and most preferably to a degree of 75.0 to 100.0 % using a neutralizing agent containing ions of zinc and, optionally other alkali metals and/or alkaline earth metals. In one embodiment, the acidic sites of the polymeric salt of zinc are neutralized using a neutralizing agent containing only zinc. Neutralized polyacrylates and/or polymethacrylates with an average molecular weight of not more than 50 000, preferably with an average molecular weight in the range from 1 000 to 25 000 and more preferably in the range from 3 000 to 12 000 are especially suitable.

In a preferred embodiment, the at least one water soluble or water dispersable source of magnesium ions and/or zinc ions is preferably at least one water soluble or water dispersable source of zinc ions. That is to say, the first buffer component is preferably a zinc ion-comprising material.

It is a further requirement that the buffer composition comprises at least one alkali carbonate and/or at least one alkali bicarbonate as a second buffer component.

The term "at least one" alkali carbonate in the meaning of the present invention means that the alkali carbonate comprises, preferably consists of, one or more alkali carbonate(s).

In one embodiment of the present invention, the alkali carbonate comprises, preferably consists of, one alkali carbonate. Alternatively, the alkali carbonate comprises, preferably consists of, two or more alkali carbonates. For example, the alkali carbonate comprises, preferably consists of, two or three alkali carbonates. Preferably, the alkali carbonate comprises, preferably consists of, one alkali carbonate.

The term "at least one" alkali bicarbonate in the meaning of the present invention means that the alkali bicarbonate comprises, preferably consists of, one or more alkali bicarbonate(s).

In one embodiment of the present invention, the alkali bicarbonate comprises, preferably consists of, one alkali bicarbonate. Alternatively, the alkali bicarbonate comprises, preferably consists of, two or more alkali bicarbonates. For example, the alkali bicarbonate comprises, preferably consists of, two or three alkali bicarbonates. Preferably, the alkali bicarbonate comprises, preferably consists of, one alkali bicarbonate.

It is appreciated that the at least one alkali carbonate and/or at least one alkali bicarbonate of the instant buffer composition can be any material comprising, preferably consisting of, alkali carbonate and/or alkali bicarbonate.

For example, the at least one alkali carbonate and/or at least one alkali bicarbonate is at least one alkali carbonate and at least one alkali bicarbonate, i.e. a mixture of the at least one alkali carbonate and at least one alkali bicarbonate. Alternatively, the at least one alkali carbonate and/or at least one alkali bicarbonate is at least one alkali carbonate or at least one alkali bicarbonate.

In one embodiment, the at least one alkali carbonate is selected from the group consisting of sodium carbonate, potassium carbonate, lithium carbonate, and mixtures thereof. Preferably, the at least one alkali carbonate is selected from the group consisting of sodium carbonate and/or lithium carbonate. More preferably, the at least one alkali carbonate is lithium carbonate (CAS NO. 554-13-2).

Additionally or alternatively, the at least one alkali bicarbonate is selected from the group consisting of sodium bicarbonate, potassium bicarbonate, lithium bicarbonate, and mixtures thereof. Preferably, the at least one alkali bicarbonate is selected from the group consisting of sodium bicarbonate and/or lithium bicarbonate. More preferably, the at least one alkali bicarbonate is lithium bicarbonate.

In one embodiment, the at least one alkali carbonate and/or at least one alkali bicarbonate is a mixture of at least one alkali carbonate and at least one alkali bicarbonate. For example, the at least one alkali carbonate and at least one alkali bicarbonate is a mixture of sodium carbonate and sodium bicarbonate, or a mixture of potassium carbonate and potassium bicarbonate, or a mixture of lithium carbonate and lithium bicarbonate. Preferably, the at least one alkali carbonate and at least one alkali bicarbonate is a mixture of sodium carbonate and sodium bicarbonate or a mixture of lithium carbonate and lithium bicarbonate. More preferably, the at least one alkali carbonate and at least one alkali bicarbonate is a mixture of lithium carbonate and lithium bicarbonate.

In view of the above, it is preferred that the first buffer component is at least one water soluble or water dispersable source of zinc ions being selected from the group comprising zinc carbonate, zinc oxide, zinc hydroxide, zinc phosphate, and mixtures thereof, and the second buffer component is at least one alkali carbonate being selected from the group consisting of sodium carbonate, potassium carbonate, lithium carbonate, and mixtures thereof.

In a preferred embodiment, the first buffer component is at least one water soluble or water dispersable source of zinc ions being zinc oxide, and the second buffer component is at least one alkali carbonate being sodium carbonate and/or lithium carbonate. For example, the first buffer component is at least one water soluble or water dispersable source of zinc ions being zinc oxide, and the second buffer component is at least one alkali carbonate being sodium carbonate and lithium carbonate. Alternatively, the first buffer component is at least one water soluble or water dispersable source of zinc ions being zinc oxide, and the second buffer component is at least one alkali carbonate being sodium carbonate or lithium carbonate.

Thus, it is preferred that the first buffer component is zinc oxide and the second buffer component is lithium carbonate.

Additionally or alternatively, the first buffer component is at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being selected from the group consisting of sodium bicarbonate, potassium bicarbonate, lithium bicarbonate, and mixtures thereof.

In a preferred embodiment, the first buffer component is at least one water soluble or water dispersable source of zinc ions being zinc oxide, and the second buffer component is at least one alkali bicarbonate being sodium bicarbonate and/or lithium bicarbonate. For example, the first buffer component is at least one water soluble or water dispersable source of zinc ions being zinc oxide, and the second buffer component is at least one alkali bicarbonate being sodium bicarbonate and lithium bicarbonate. Alternatively, the first buffer component is at least one water soluble or water dispersable source of zinc ions being zinc oxide, and the second buffer component is at least one alkali bicarbonate being sodium bicarbonate or lithium bicarbonate.

In a preferred embodiment, the first buffer component is zinc oxide and the second buffer component is lithium bicarbonate.

Alternatively the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof and the second buffer component is at least one alkali carbonate being selected from the group consisting of sodium carbonate, potassium carbonate, lithium carbonate, and mixtures thereof.

In a preferred embodiment, the first buffer component is at least one water soluble or water dispersable source of magnesium ions being magnesium oxide and/or magnesium hydroxide, and the second buffer component is at least one alkali carbonate being sodium carbonate and/or lithium carbonate. For example, the first buffer component is at least one water soluble or water dispersable source of magnesium ions being magnesium oxide, and the second buffer component is at least one alkali carbonate being sodium carbonate and lithium carbonate. Alternatively, the first buffer component is at least one water soluble or water dispersable source of magnesium ions being magnesium oxide, and the second buffer component is at least one alkali carbonate being sodium carbonate or lithium carbonate.

Thus, it is preferred that the first buffer component is magnesium oxide and the second buffer component is lithium carbonate.

Additionally or alternatively, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof and the second buffer component is at least one alkali bicarbonate being selected from the group consisting of sodium bicarbonate, potassium bicarbonate, lithium bicarbonate, and mixtures thereof.

In a preferred embodiment, the first buffer component is at least one water soluble or water dispersable source of magnesium ions being magnesium oxide and/or magnesium hydroxide, and the second buffer component is at least one alkali bicarbonate being sodium bicarbonate and/or lithium bicarbonate. For example, the first buffer component is at least one water soluble or water dispersable source of magnesium ions being magnesium oxide, and the second buffer component is at least one alkali bicarbonate being sodium bicarbonate and lithium bicarbonate. Alternatively, the first buffer component is at least one water soluble or water dispersable source of magnesium ions being magnesium oxide, and the second buffer component is at least one alkali bicarbonate being sodium bicarbonate or lithium bicarbonate.

In a preferred embodiment, the first buffer component is magnesium oxide and the second buffer component is lithium bicarbonate.

It is a further requirements of the present buffer composition that the molar ratio of the first buffer component to the second buffer component [Mg and/or Zn ions / carbonate and/or bicarbonate ions] is from 10 000:1 to 1:10 000. Preferably, the molar ratio of the first buffer component to the second buffer component [Mg and/or Zn ions / carbonate and/or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

For example, the molar ratio of the first buffer component to the second buffer component [Mg and Zn ions / carbonate and/or bicarbonate ions] is from 10 000:1 to 1:10 000. Preferably, the molar ratio of the first buffer component to the second buffer component [Mg and Zn ions / carbonate and/or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the molar ratio of the first buffer component to the second buffer component [Mg or Zn ions / carbonate and/or bicarbonate ions] is from 10 000:1 to 1:10 000. Preferably, the molar ratio of the first buffer component to the second buffer component [Mg or Zn ions / carbonate and/or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

For example, the molar ratio of the first buffer component to the second buffer component [Mg and Zn ions / carbonate or bicarbonate ions] is from 10 000:1 to 1:10 000. Preferably, the molar ratio of the first buffer component to the second buffer component [Mg and Zn ions / carbonate or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the molar ratio of the first buffer component to the second buffer component [Mg or Zn ions / carbonate or bicarbonate ions] is from 10 000:1 to 1:10 000. Preferably, the molar ratio of the first buffer component to the second buffer component [Mg or Zn ions / carbonate or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

Alternatively, the molar ratio of the first buffer component to the second buffer component [Mg and Zn ions / carbonate and bicarbonate ions] is from 10 000:1 to 1:10 000. Preferably, the molar ratio of the first buffer component to the second buffer component [Mg and Zn ions / carbonate and bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the molar ratio of the first buffer component to the second buffer component [Mg or Zn ions / carbonate and bicarbonate ions] is from 10 000:1 to 1:10 000. Preferably, the molar ratio of the first buffer component to the second buffer component [Mg or Zn ions / carbonate and bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

It is appreciated that the molar ratio throughout the present description does not refer to the complete compound of the first and second buffer components but rather refers to the corresponding Mg and/or Zn ions and carbonate and/or bicarbonate ions only.

In case the buffer composition comprises Mg and Zn ions, it is appreciated that the molar ratio of Mg ions to Zn ions throughout the present invention is from 20:1 to 1:20, preferably from 10:1 to 1:10, even more preferably from 5:1 to 1:5 and most preferably from 2:1 to 1:2, e.g. about 1:1.

If the buffer composition comprises carbonate and bicarbonate ions, it is appreciated that the molar ratio of carbonate ions to bicarbonate ions throughout the present invention is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In one embodiment, the first buffer component is at least one water soluble or water dispersable source of zinc ions being zinc oxide, and the second buffer component is at least one alkali carbonate being sodium carbonate and/or lithium carbonate, wherein the molar ratio of the first buffer component to the second buffer component [Zn ions / carbonate ions] is from 10 000:1 to 1:10 000.

For example, the first buffer component is at least one water soluble or water dispersable source of zinc ions being zinc oxide, and the second buffer component is at least one alkali carbonate being sodium carbonate and lithium carbonate, wherein the molar ratio of the first buffer component to the second buffer component [Zn ions / carbonate ions] is from 10 000:1 to 1:10 000. Alternatively, the first buffer component is at least one water soluble or water dispersable source of zinc ions being zinc oxide, and the second buffer component is at least one alkali carbonate being sodium carbonate or lithium carbonate, the molar ratio of the first buffer component to the second buffer component [Zn ions / carbonate ions] is from 10 000:1 to 1:10 000.

Thus, it is preferred that the first buffer component is zinc oxide and the second buffer component is lithium carbonate, wherein the molar ratio of the first buffer component to the second buffer component [Zn ions / carbonate ions] is from 10 000:1 to 1:10 000.

In a preferred embodiment, the first buffer component is at least one water soluble or water dispersable source of zinc ions being zinc oxide, and the second buffer component is at least one alkali carbonate being sodium carbonate and lithium carbonate, wherein the molar ratio of the first buffer component to the second buffer component [Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the first buffer component is at least one water soluble or water dispersable source of zinc ions being zinc oxide, and the second buffer component is at least one alkali carbonate being sodium carbonate or lithium carbonate, wherein the molar ratio of the first buffer component to the second buffer component [Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

Thus, it is preferred that the first buffer component is zinc oxide and the second buffer component is lithium carbonate, wherein the molar ratio of the first buffer component to the second buffer component [Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In one embodiment, the first buffer component is at least one water soluble or water dispersable source of zinc ions being zinc oxide, and the second buffer component is at least one alkali bicarbonate being sodium bicarbonate and/or lithium bicarbonate, wherein the molar ratio of the first buffer component to the second buffer component [Zn ions / bicarbonate ions] is from 10 000:1 to 1:10 000.

For example, the first buffer component is at least one water soluble or water dispersable source of zinc ions being zinc oxide, and the second buffer component is at least one alkali bicarbonate being sodium bicarbonate and lithium bicarbonate, wherein the molar ratio of the first buffer component to the second buffer component [Zn ions / bicarbonate ions] is from 10 000:1 to 1:10 000. Alternatively, the first buffer component is at least one water soluble or water dispersable source of zinc ions being zinc oxide, and the second buffer component is at least one alkali bicarbonate being sodium bicarbonate or lithium bicarbonate, the molar ratio of the first buffer component to the second buffer component [Zn ions / bicarbonate ions] is from 10 000:1 to 1:10 000.

Thus, it is preferred that the first buffer component is zinc oxide and the second buffer component is lithium bicarbonate, wherein the molar ratio of the first buffer component to the second buffer component [Zn ions / bicarbonate ions] is from 10 000:1 to 1:10 000.

In a preferred embodiment, the first buffer component is at least one water soluble or water dispersable source of zinc ions being zinc oxide, and the second buffer component is at least one alkali bicarbonate being sodium bicarbonate and lithium bicarbonate, wherein the molar ratio of the first buffer component to the second buffer component [Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the first buffer component is at least one water soluble or water dispersable source of zinc ions being zinc oxide, and the second buffer component is at least one alkali bicarbonate being sodium bicarbonate or lithium bicarbonate, wherein the molar ratio of the first buffer component to the second buffer component [Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

For example, the first buffer component is zinc oxide and the second buffer component is lithium bicarbonate, wherein the molar ratio of the first buffer component to the second buffer component [Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In an alternative embodiment, the first buffer component is at least one water soluble or water dispersable source of magnesium ions being magnesium oxide, and the second buffer component is at least one alkali carbonate being sodium carbonate and/or lithium carbonate, wherein the molar ratio of the first buffer component to the second buffer component [Mg ions / carbonate ions] is from 10 000:1 to 1:10 000.

For example, the first buffer component is at least one water soluble or water dispersable source of magnesium ions being magnesium oxide, and the second buffer component is at least one alkali carbonate being sodium carbonate and lithium carbonate, wherein the molar ratio of the first buffer component to the second buffer component [Mg ions / carbonate ions] is from 10 000:1 to 1:10 000. Alternatively, the first buffer component is at least one water soluble or water dispersable source of magnesium ions being magnesium oxide, and the second buffer component is at least one alkali carbonate being sodium carbonate or lithium carbonate, the molar ratio of the first buffer component to the second buffer component [Mg ions / carbonate ions] is from 10 000:1 to 1:10 000.

Thus, it is preferred that the first buffer component is magnesium oxide and the second buffer component is lithium carbonate, wherein the molar ratio of the first buffer component to the second buffer component [Mg ions / carbonate ions] is from 10 000:1 to 1:10 000.

In a preferred embodiment, the first buffer component is at least one water soluble or water dispersable source of magnesium ions being magnesium oxide, and the second buffer component is at least one alkali carbonate being sodium carbonate and lithium carbonate, wherein the molar ratio of the first buffer component to the second buffer component [Mg ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the first buffer component is at least one water soluble or water dispersable source of magnesium ions being magnesium oxide, and the second buffer component is at least one alkali carbonate being sodium carbonate or lithium carbonate, wherein the molar ratio of the first buffer component to the second buffer component [Mg ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

Thus, it is preferred that the first buffer component is magnesium oxide and the second buffer component is lithium carbonate, wherein the molar ratio of the first buffer component to the second buffer component [Mg ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In one embodiment, the first buffer component is at least one water soluble or water dispersable source of magnesium ions being magnesium oxide, and the second buffer component is at least one alkali bicarbonate being sodium bicarbonate and/or lithium bicarbonate, wherein the molar ratio of the first buffer component to the second buffer component [Mg ions / bicarbonate ions] is from 10 000:1 to 1:10 000.

For example, the first buffer component is at least one water soluble or water dispersable source of magnesium ions being magnesium oxide, and the second buffer component is at least one alkali bicarbonate being sodium bicarbonate and lithium bicarbonate, wherein the molar ratio of the first buffer component to the second buffer component [Mg ions / bicarbonate ions] is from 10 000:1 to 1:10 000. Alternatively, the first buffer component is at least one water soluble or water dispersable source of magnesium ions being magnesium oxide, and the second buffer component is at least one alkali bicarbonate being sodium bicarbonate or lithium bicarbonate, the molar ratio of the first buffer component to the second buffer component [Mg ions / bicarbonate ions] is from 10 000:1 to 1:10 000.

Thus, it is preferred that the first buffer component is magnesium oxide and the second buffer component is lithium bicarbonate, wherein the molar ratio of the first buffer component to the second buffer component [Mg ions / bicarbonate ions] is from 10 000:1 to 1:10 000.

In a preferred embodiment, the first buffer component is at least one water soluble or water dispersable source of magnesium ions being magnesium oxide, and the second buffer component is at least one alkali bicarbonate being sodium bicarbonate and lithium bicarbonate, wherein the molar ratio of the first buffer component to the second buffer component [Mg ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. Alternatively, the first buffer component is at least one water soluble or water dispersable source of magnesium ions being magnesium oxide, and the second buffer component is at least one alkali bicarbonate being sodium bicarbonate or lithium bicarbonate, wherein the molar ratio of the first buffer component to the second buffer component [Mg ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

For example, the first buffer component is magnesium oxide and the second buffer component is lithium bicarbonate, wherein the molar ratio of the first buffer component to the second buffer component [Mg ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

As already mentioned above, the first buffer component can be at least one water soluble or water dispersable source of magnesium ions and zinc ions. That is to say, the buffer composition comprises at least one water soluble or water dispersable source of magnesium ions and at least one water soluble or water dispersable source of zinc ions as the first buffer component in combination with at least one alkali carbonate and/or at least one alkali bicarbonate as the second buffer component.

For example, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate being sodium carbonate and/or lithium carbonate and/or at least one alkali bicarbonate being sodium bicarbonate and/or lithium bicarbonate.

Preferably, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate being sodium carbonate and/or lithium carbonate. Preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali carbonate being lithium carbonate.

Alternatively, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being sodium bicarbonate and/or lithium bicarbonate. Preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali bicarbonate being lithium bicarbonate.

In one embodiment, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate is sodium carbonate and/or lithium carbonate, wherein the molar ratio of the first buffer component to the second buffer component [Mg and Zn ions / carbonate ions] is from 10 000:1 to 1 :10 000.

Preferably, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate being sodium carbonate and/or lithium carbonate, wherein the molar ratio of the first buffer component to the second buffer component [Mg and Zn ions / carbonate ions] is from 10 000:1 to 1:10 000. More preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali carbonate being lithium carbonate, wherein the molar ratio of the first buffer component to the second buffer component [Mg and Zn ions / carbonate ions] is from 10 000:1 to 1:10 000.

In this embodiment, the molar ratio of Mg ions to Zn ions is preferably from 20:1 to 1:20, more preferably from 10:1 to 1:10, even more preferably from 5:1 to 1:5 and most preferably from 2:1 to 1:2, e.g. about 1:1.

In one embodiment, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate being sodium carbonate and/or lithium carbonate, wherein the molar ratio of the first buffer component to the second buffer component [Mg and Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

Preferably, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali carbonate being sodium carbonate and/or lithium carbonate, wherein the molar ratio of the first buffer component to the second buffer component [Mg and Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. More preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali carbonate being lithium carbonate, wherein the molar ratio of the first buffer component to the second buffer component [Mg and Zn ions / carbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In this embodiment, the molar ratio of Mg ions to Zn ions is preferably from 20:1 to 1:20, more preferably from 10:1 to 1:10, even more preferably from 5:1 to 1:5 and most preferably from 2:1 to 1:2, e.g. about 1:1.

In one embodiment, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, and at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being sodium bicarbonate and/or lithium bicarbonate, wherein the molar ratio of the first buffer component to the second buffer component [Mg and Zn ions / bicarbonate ions] is from 10 000:1 to 1:10 000.

Preferably, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being sodium bicarbonate and/or lithium bicarbonate, wherein the molar ratio of the first buffer component to the second buffer component [Mg and Zn ions / bicarbonate ions] is from 10 000:1 to 1:10 000. More preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali bicarbonate being lithium bicarbonate, wherein the molar ratio of the first buffer component to the second buffer component [Mg and Zn ions / bicarbonate ions] is from 10 000:1 to 1:10 000.

In one embodiment, the first buffer component is at least one water soluble or water dispersable source of magnesium ions being selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, and at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being sodium bicarbonate and/or lithium bicarbonate, wherein the molar ratio of the first buffer component to the second buffer component [Mg and Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In this embodiment, the molar ratio of Mg ions to Zn ions is preferably from 20:1 to 1:20, more preferably from 10:1 to 1:10, even more preferably from 5:1 to 1:5 and most preferably from 2:1 to 1:2, e.g. about 1:1.

Preferably, the first buffer component is at least one water soluble or water dispersable source of magnesium ions being magnesium oxide and/or magnesium hydroxide, and at least one water soluble or water dispersable source of zinc ions being selected from the group comprising zinc carbonate, zinc oxide, zinc hydroxide, zinc phosphate, and mixtures thereof, and the second buffer component is at least one alkali bicarbonate being sodium bicarbonate and/or lithium bicarbonate, wherein the molar ratio of the first buffer component to the second buffer component [Mg and Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1. More preferably, the first buffer component is at least one water soluble or water dispersable source of magnesium ions being magnesium oxide and/or magnesium hydroxide, and at least one water soluble or water dispersable source of zinc ions being zinc oxide, and the second buffer component is at least one alkali bicarbonate being lithium bicarbonate, wherein the molar ratio of the first buffer component to the second buffer component [Mg and Zn ions / bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

In this embodiment, the molar ratio of Mg ions to Zn ions is preferably from 20:1 to 1:20, more preferably from 10:1 to 1:10, even more preferably from 5:1 to 1:5 and most preferably from 2:1 to 1:2, e.g. about 1:1.

It is to be noted that the molar amount of the magnesium ions and zinc ions for the first buffer component refers to the sum of the molar amount of the magnesium ions and zinc ions.

Furthermore, it is to be noted that the molar amount of the alkali carbonate and alkali bicarbonate for the second buffer component refers to the sum of the molar amount of the carbonate ions and bicarbonate ions.

It is appreciated that the buffer composition can further comprise at least one water soluble or water dispersable source sodium ions. This embodiment may be advantageous in order to further improve the buffer capacity of the buffer composition.

Thus, it is preferred that the present buffer composition comprises the first buffer component being at least one water soluble or water dispersable source of magnesium ions and/or zinc ions and the second buffer component being at least one alkali carbonate and/or at least one alkali bicarbonate in combination with at least one water soluble or water dispersable source of sodium ions.

In one embodiment, the buffer composition thus comprises the first buffer component being at least one water soluble or water dispersable source of magnesium ions and zinc ions and the second buffer component being at least one alkali carbonate and/or at least one alkali bicarbonate in combination with at least one water soluble or water dispersable source of sodium ions. Alternatively, the buffer composition comprises the first buffer component being at least one water soluble or water dispersable source of magnesium ions or zinc ions and the second buffer component being at least one alkali carbonate and/or at least one alkali bicarbonate in combination with at least one water soluble or water dispersable source of sodium ions. Preferably, the buffer composition comprises the first buffer component being at least one water soluble or water dispersable source of zinc ions and the second buffer component being at least one alkali carbonate and/or at least one alkali bicarbonate in combination with at least one water soluble or water dispersable source of sodium ions.

In one embodiment, the buffer composition comprises the first buffer component being at least one water soluble or water dispersable source of magnesium ions and/or zinc ions and the second buffer component being at least one alkali carbonate and at least one alkali bicarbonate in combination with at least one water soluble or water dispersable source of sodium ions. Alternatively, the buffer composition comprises the first buffer component being at least one water soluble or water dispersable source of magnesium ions and/or zinc ions and the second buffer component being at least one alkali carbonate or at least one alkali bicarbonate in combination with at least one water soluble or water dispersable source of sodium ions. Preferably, the buffer composition comprises the first buffer component being at least one water soluble or water dispersible source of magnesium ions and/or zinc ions and the second buffer component being at least one alkali carbonate in combination with at least one water soluble or water dispersible source of sodium ions. For example, the buffer composition comprises the first buffer component being at least one water soluble or water dispersible source of magnesium ions or zinc ions and the second buffer component being at least one alkali carbonate or at least one alkali bicarbonate in combination with at least one water soluble or water dispersible source of sodium ions. Preferably, the buffer composition comprises the first buffer component being at least one water soluble or water dispersable source of zinc ions and the second buffer component being at least one alkali carbonate in combination with at least one water soluble or water dispersable source of sodium ions.

The term source of "at least one" water soluble or water dispersable source of sodium ions in the meaning of the present invention means that the source comprises, preferably consists of, one or more water soluble or water dispersable source(s) of sodium ions.

In one embodiment of the present invention, the water soluble or water dispersable source of sodium ions comprises, preferably consists of, one water soluble or water dispersable source of sodium ions. Alternatively, the water soluble or water dispersable source of sodium ions comprises, preferably consists of, two or more water soluble or water dispersable sources of sodium ions. For example, the water soluble or water dispersable source of sodium ions comprises, preferably consists of, two or three water soluble or water dispersable sources of sodium ions. Preferably, the water soluble or water dispersable source of sodium ions comprises, preferably consists of, two or more water soluble or water dispersable sources of sodium ions

It is appreciated that the at least one water soluble or water dispersable source of sodium ions of the present buffer composition can be any material comprising, preferably consisting of, sodium ions as ions.

It is appreciated that the at least one source of sodium ions is water soluble or water dispersable.

Accordingly, the term "water soluble" or "soluble in water" in the meaning of the present invention refers to systems in which the source of sodium ions forms a solution with water, i.e. the particles of the at least one source of sodium ions are dissolved in the solvent. Alternatively, the term "water dispersable" or "dispersable in water" in the meaning of the present invention refers to systems in which only a part of the source of sodium ions forms a solution with water, i.e. only a part of the particles of the at least one source of sodium ions are dissolved in the solvent.

The term "source" of sodium ions in the meaning of the present invention refers to a compound that comprises, preferably consists of, sodium ions as ions, i.e. sodium ions.

In one embodiment of the present invention, the at least one water soluble or water dispersable source of sodium ions is preferably provided in the form of at least one sodium salt. Preferably the ionic group of the at least one sodium salt is selected from the group comprising oxide, chloride, hydroxide, phosphate, citrate, maleate, acetate, lactate and mixtures thereof. In particular, the at least one sodium salt is selected from sodium carbonate, sodium chloride, sodium hydroxide, sodium phosphate, sodium citrate, sodium maleate, sodium acetate and sodium lactate; polymeric salts of sodium and mixtures thereof.

For example, the at least one water soluble or water dispersable source of sodium ions is preferably sodium oxide and/or sodium hydroxide, preferably sodium hydroxide..

Additionally or alternatively, the at least one water soluble or water dispersable source of sodium ions can be present as a polymeric salt of sodium, such as acrylic homopolymers, acrylic copolymers such as copolymers of acrylic acid and maleic acid and/or acrylamide, polyphosphates and mixtures thereof having multiple acidic sites which can be partially or totally neutralised with sodium ions. The polymeric salt of sodium is preferably selected from Li₂Na₂polyphosphate, lithium-sodium hexamethaphosphate or sodium polyacrylate.

The polymeric salt of sodium is preferably partially or completely neutralized, preferably to a degree of 5.0 to 100.0 %, preferably to a degree of 25.0 to 100.0 % and most preferably to a degree of 75.0 to 100.0 % using a neutralizing agent containing ions of sodium and, optionally other alkali metals and/or alkaline earth metals. In one embodiment, the acidic sites of the polymeric salt of sodium are neutralized using a neutralizing agent containing only sodium. Neutralized polyacrylates and/or polymethacrylates with an average molecular weight of not more than 50 000 Da, preferably with an average molecular weight in the range from 1 000 to 25 000 Da and more preferably in the range from 3 000 to 12 000 Da are especially suitable.

If the buffer composition further comprises at least one water soluble or water dispersable source of sodium ions, the molar ratio of the at least one water soluble or water dispersable source of magnesium ions and/or zinc ions to the at least one water soluble or water dispersable source of sodium ions [Mg and/or Zn ions/Na ions] is preferably from 10 000:1 to 1:10 000, more preferably from 1 000:1 to 1:1 000, even more preferably from 100:1 to 1:100, still more preferably from 20:1 to 1:20, still even more preferably from 10:1 to 1:10 and most preferably from 2:1 to 1:2, e.g. about 1:1.

If the buffer composition comprises Mg and Zn ions, the molar ratio of Mg ions to Zn ions is from 20:1 to 1:20, preferably from 10:1 to 1:10, even more preferably from 5:1 to 1:5 and most preferably from 2:1 to 1:2, e.g. about 1:1.

Additionally or alternatively, the molar ratio of the at least one alkali carbonate and/or at least one alkali bicarbonate to the at least one water soluble or water dispersable source of sodium ions [carbonate and/or bicarbonate ions / Na ions] is preferably from 10 000:1 to 1:10 000, more preferably from 1 000:1 to 1:1 000, even more preferably from 100:1 to 1:100, still more preferably from 20:1 to 1:20, still even more preferably from 10:1 to 1:10 and most preferably from 2:1 to 1:2, e.g. about 1:1.

If the buffer composition comprises carbonate and bicarbonate ions, the molar ratio of carbonate ions to bicarbonate ions is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

The buffer composition is preferably added to an aqueous preparation such as a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation, in order to stabilize the aqueous preparation against pH changes. As the buffer composition can be more evenly distributed in the aqueous preparation, it is preferred adding the buffer composition in form of a solution, dispersion, suspension or paste. Thus, the buffer composition preferably comprises a solvent. The solvent may be an organic solvent and/or water. Preferably, the buffer composition comprises an organic solvent and water. Alternatively, the buffer composition comprises an organic solvent or water, preferably water. Thus, if the buffer composition is provided in form of a solution, dispersion, suspension or paste, it is preferably an aqueous buffer composition.

The term "aqueous" preparation or buffer composition refers to a system, wherein the liquid phase of the preparation or composition comprises, preferably consists of, water. However, said term does not exclude that the aqueous preparation or composition comprises minor amounts of an organic solvent selected from the group comprising alcohols such as methanol, ethanol, isopropanol, carbonyl-group containing solvents such as ketones, e.g. acetone or aldehydes, esters such as isopropyl acetate, carboxylic acids such as formic acid, sulphoxides such as dimethyl sulphoxide and mixtures thereof. If the aqueous preparation or composition comprises an organic solvent, the aqueous preparation or composition comprises the organic solvent in an amount up to 40.0 wt.-% preferably from 1.0 to 30.0 wt.-% and most preferably from 1.0 to 25.0 wt.-%, based on the total weight of the liquid phase of the aqueous preparation or composition. For example, the liquid phase of the aqueous preparation or composition consists of water. If the liquid phase of the aqueous preparation or composition consists of water, the water to be used can be any water available such as tap water and/or deionised water.

Alternatively, the buffer composition comprises an organic solvent. In this case, the liquid phase of the composition comprises, preferably consists of, the organic solvent, preferably the organic solvent is selected from the group comprising alcohols such as methanol, ethanol, isopropanol, carbonyl-group containing solvents such as ketones, e.g. acetone or aldehydes, esters such as isopropyl acetate, carboxylic acids such as formic acid, sulphoxides such as dimethyl sulphoxide and mixtures thereof. However, said term does not exclude that the composition comprises minor amounts of an water. If the composition comprises water, the composition comprises water in an amount up to 40.0 wt.-% preferably from 1.0 to 30.0 wt.-% and most preferably from 1.0 to 25.0 wt.-%, based on the total weight of the liquid phase of the composition. For example, the liquid phase of the composition consists of the organic solvent.

The term "solution" in the meaning of the present invention refers to a buffer composition in which no discrete solid particles are observed in the solvent, i.e. a solution with water and/or organic solvent is formed, wherein the first buffer component in combination with the second buffer component and optionally the at least one water soluble or water dispersable source of sodium ions are dissolved in the solvent.

The term "dispersion" or "suspension" in the meaning of the present invention refers to a buffer composition, wherein at least a part of the first buffer component in combination with the second buffer component and optionally the at least one water soluble or water dispersable source of sodium ions are present as insoluble solids in the solvent. The dispersion or suspension preferably has a solids content up to 80.0 wt.-% preferably from 0.5 to 80.0 wt.-% and most preferably from 1.0 to 60.0 wt.-%, based on the total weight of the dispersion or suspension.

In an alternative embodiment, the buffer composition is in form of a powder, granules, pellets or tablets. That is to say, the buffer composition in form of a powder, granules, pellets or tablets is free of a solvent. This may be advantageous if the buffer composition is stored or transported as it requires less space and weight. Furthermore, the article to be treated with the buffer composition in form of a powder, granules, pellets or tablets, is not diluted by using a solvent free buffer composition, which may be advantageous in cases where no increase of the solvent amount is desired or possible.

In a preferred embodiment, the buffer composition is in form of a powder.

Such products are well known in the art and the skilled person is well aware of methods for producing same such that there is no need to describe them in more detail in the present application.

It is to be noted that the buffer components described herein are preferably the only components in the buffer composition. Thus, the buffer composition is preferably free of buffer components comprising ammonia, methylamine, dimethylamine, trimethylamine, diethylamine, propylamine, butylamine, hexylamine, amino-2-methylpropanol (AMP), monoethanolamine (MEA), monoisopropanolamine (MIPA), triethylenetetramine (TETA), calcium hydroxide and mixtures thereof.

In a preferred embodiment, the buffer composition is free of amine-based buffer components, monoalcohol-based buffer components, primary alkanol amine-based buffer components, hydroxide-based buffer components and mixtures thereof.

In view of this, the buffer composition does not deteriorate the optical properties of the preparation and does not adversely affect the smell of the preparation.

Thus, the buffer composition comprises, preferably consists of,
a) the first buffer component being at least one water soluble or water dispersable source of magnesium ions and/or zinc ions, and
b) the second buffer component being at least one alkali carbonate and/or at least one alkali bicarbonate, and
c) optionally at least one water soluble or water dispersable source of sodium ions, and
d) optionally a solvent, preferably an organic solvent and/or water,
wherein the molar ratio of the first buffer component to the second buffer component [Mg and/or Zn ions / carbonate and/or bicarbonate ions] is from 10 000:1 to 1:10 000.

As already mentioned above, it has been found that the present buffer composition is capable of maintaining the pH of an aqueous preparation or a solid article equal to or below 12. Preferably, the buffer composition of the present invention is capable of maintaining the pH of an aqueous preparation or a solid article in the range from 4 to 12, more preferably from 6 to 11.5 and most preferably from 8.5 to 10.5.

### Articles, processes and uses

The buffer composition of the present invention is used to maintaining the pH of an article, such as an aqueous preparation or a solid article, equal to or below 12. Thus, the article can be any kind of article in need of a pH stabilization.

More precisely, the article comprises the buffer composition.

It is appreciated that each of the first and the second buffer components are present in the article in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the article. That is to say, the first buffer component is present in the article in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the article, and the second buffer component is present in the article in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the article.

It is to be noted that the amount of the first and the second buffer components in the article can be chosen independently from each other. Thus, the amount of the first buffer component in the article can be different to the amount of the second buffer component in the article. In another embodiment, the amount of the first buffer component in the article is similar or identical to the amount of the second buffer component in the article.

If the article further comprises at least one water soluble or water dispersable source of sodium ions, the at least one water soluble or water dispersable source of sodium ions is present in the article in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the article.

It is appreciated that, if it is not indicated otherwise, the term "ppm" is calculated relative to the weight of the article, i.e. the aqueous preparation or the solid article.

In one embodiment, the article can be an aqueous preparation. The aqueous preparation is preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation.

In one embodiment, the first and/or the second buffer component(s) is/are present in a supersaturated state in the aqueous preparation. For example, the first or the second buffer component is present in a supersaturated state in the aqueous preparation. Alternatively, the first and the second buffer components are present in a supersaturated state in the aqueous preparation.

The term "supersaturated" refers to the amount of the corresponding buffer component in the aqueous preparation exceeding the amount specified by the value equilibrium solubility at room temperature and atmospheric pressure.

In order to obtain a sufficient buffer capacity, it is advantageous that at least the first buffer component is present in a supersaturated state in the aqueous preparation. In a preferred embodiment, the first and the second buffer components are present in a supersaturated state in the aqueous preparation.

It is appreciated that each of the first and the second buffer components are present in the aqueous preparation in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation. That is to say, the first buffer component is present in the aqueous preparation in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and the second buffer component is present in the aqueous preparation in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation.

Preferably, the first buffer component is present in a supersaturated state in the aqueous preparation.

For example, the first buffer component is present in a supersaturated state in the aqueous preparation, whereas the second buffer component is present in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation.

If the aqueous preparation further comprises at least one water soluble or water dispersable source of sodium ions, the at least one water soluble or water dispersable source of sodium ions is present in the aqueous preparation in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation.

Thus, it is preferred that the aqueous preparation comprises the buffer composition such that
a) the first buffer component being at least one water soluble or water dispersable source of magnesium ions and/or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate and/or bicarbonate ions] is from 10 000:1 to 1:10 000.

The first buffer component can be at least one water soluble or water dispersable source of magnesium ions and zinc ions. In this case, the aqueous preparation comprises the buffer composition such that
a) the first buffer component being at least one water soluble or water dispersable source of magnesium ions and zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and Zn ions / carbonate and/or bicarbonate ions] is from 10 000:1 to 1:10 000.

The first buffer component can be at least one water soluble or water dispersable source of magnesium ions or zinc ions. In this case, the aqueous preparation comprises the buffer composition such that
a) the first buffer component being at least one water soluble or water dispersable source of magnesium ions or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg or Zn ions / carbonate and/or bicarbonate ions] is from 10 000:1 to 1:10 000.

The second buffer component can be at least one alkali carbonate and at least one alkali bicarbonate. In this case, the aqueous preparation comprises the buffer composition such that
a) the first buffer component being at least one water soluble or water dispersable source of magnesium ions and/or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate and bicarbonate ions] is from 10 000:1 to 1:10 000.

The second buffer component can be at least one alkali carbonate or at least one alkali bicarbonate. In this case, the aqueous preparation comprises the buffer composition such that
a) the first buffer component being at least one water soluble or water dispersable source of magnesium ions and/or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate or bicarbonate ions] is from 10 000:1 to 1:10 000.

The first buffer component can be at least one water soluble or water dispersable source of magnesium ions and zinc ions and the second buffer component can be at least one alkali carbonate and at least one alkali bicarbonate. In this case, the aqueous preparation comprises the buffer composition such that
a) the first buffer component being at least one water soluble or water dispersable source of magnesium ions and zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and Zn ions / carbonate and bicarbonate ions] is from 10 000:1 to 1:10 000.

The first buffer component can be at least one water soluble or water dispersable source of magnesium ions or zinc ions and the second buffer component can be at least one alkali carbonate or at least one alkali bicarbonate. In this case, the aqueous preparation comprises the buffer composition such that
a) the first buffer component being at least one water soluble or water dispersable source of magnesium ions or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg or Zn ions / carbonate or bicarbonate ions] is from 10 000:1 to 1:10 000.

For example, the aqueous preparation comprises the buffer composition such that
a) the first buffer component being at least one water soluble or water dispersable source of zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Zn ions / carbonate ions] is from 10 000:1 to 1:10 000.

If the buffer composition further comprises at least one water soluble or water dispersable source of sodium ions, the aqueous preparation comprises the buffer composition such that
a) the first buffer component being at least one water soluble or water dispersable source of magnesium ions and/or zinc ions is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate is present in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions is present in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate and/or bicarbonate ions] is from 10 000:1 to 1:10 000.

In case the first buffer component comprises at least one water soluble or water dispersable source of magnesium ions and zinc ions, the aqueous preparation comprises
a) the first buffer component being at least one water soluble or water dispersable source of magnesium ions and zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and Zn ions / carbonate and/or bicarbonate ions] is from 10 000:1 to 1:10 000.

In case the first buffer component comprises at least one water soluble or water dispersable source of magnesium ions or zinc ions, the aqueous preparation comprises
a) the first buffer component being at least one water soluble or water dispersable source of magnesium ions or zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and/or at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg or Zn ions / carbonate and/or bicarbonate ions] is from 10 000:1 to 1:10 000.

In case the second buffer component comprises at least one alkali carbonate and at least one alkali bicarbonate, the aqueous preparation comprises
a) the first buffer component being at least one water soluble or water dispersable source of magnesium ions and/or zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate and bicarbonate ions] is from 10 000:1 to 1:10 000.

In case the second buffer component comprises at least one alkali carbonate or at least one alkali bicarbonate, the aqueous preparation comprises
a) the first buffer component being at least one water soluble or water dispersable source of magnesium ions and/or zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate or at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and/or Zn ions / carbonate or bicarbonate ions] is from 10 000:1 to 1:10 000.

In case the first buffer component comprises at least one water soluble or water dispersable source of magnesium ions and zinc ions and the second buffer component comprises at least one alkali carbonate and at least one alkali bicarbonate, the aqueous preparation comprises
a) the first buffer component being at least one water soluble or water dispersable source of magnesium ions and zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate and at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg and Zn ions / carbonate and bicarbonate ions] is from 10 000:1 to 1:10 000.

In case the first buffer component comprises at least one water soluble or water dispersable source of magnesium ions or zinc ions and the second buffer component comprises at least one alkali carbonate or at least one alkali bicarbonate, the aqueous preparation comprises
a) the first buffer component being at least one water soluble or water dispersable source of magnesium ions or zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate or at least one alkali bicarbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Mg or Zn ions / carbonate or bicarbonate ions] is from 10 000:1 to 1:10 000.

For example, the aqueous preparation comprises
a) the first buffer component being at least one water soluble or water dispersable source of zinc ions in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
b) the second buffer component being at least one alkali carbonate in a supersaturated state, preferably in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the aqueous preparation,
wherein the molar ratio of the first buffer component to the second buffer component [Zn ions / carbonate ions] is from 10 000:1 to 1:10 000.

The aqueous preparation preferably comprises at least one inorganic particulate material.

The term "at least one" inorganic particulate material in the meaning of the present invention means that the inorganic particulate material comprises, preferably consists of, one or more inorganic particulate materials.

In one embodiment of the present invention, the at least one inorganic particulate material comprises, preferably consists of, one inorganic particulate material. Alternatively, the at least one inorganic particulate material comprises, preferably consists of, two or more inorganic particulate materials. For example, the at least one inorganic particulate material comprises, preferably consists of, two or three inorganic particulate material. Preferably, the at least one inorganic particulate material comprises, preferably consists of, one inorganic particulate material.

For example, the at least one inorganic particulate material is selected from the group comprising natural ground calcium carbonate, natural and/or synthetic precipitated calcium carbonate, surface-reacted calcium carbonate, dolomite, calcium sulphate, kaolin, clay, barite, talcum, quartz, mica, gypsum, aluminium hydroxide, aluminium silicate, titanium dioxide, magnesite, hydromagnesite, hydroxylapatite, perlite, sepiolite, brucite and mixtures thereof.

In one embodiment of the present invention, the at least one inorganic particulate material comprises natural ground calcium carbonate and/or synthetic precipitated calcium carbonate and/or surface-reacted calcium carbonate. Preferably, the at least one inorganic particulate material comprises natural ground calcium carbonate and/or synthetic precipitated calcium carbonate.

It is to be noted that hydromagnesite and brucite are either added as the first buffer component of the buffer composition or the at least one inorganic particulate material of the aqueous preparation. That is to say, if the aqueous preparation comprises hydromagnesite and/or brucite as the at least one inorganic particulate material, hydromagnesite and/or brucite is/are not included as the first buffer component in the buffer composition.

"Ground calcium carbonate" (GCC) in the meaning of the present invention is a calcium carbonate obtained from natural sources, such as limestone, marble or chalk, and processed through a treatment such as grinding, screening and/or fractionizing by wet and/or dry, for example by a cyclone or classifier.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water.

A "surface-reacted calcium carbonate" may feature surface-reacted GCC or PCC. A surface-reacted calcium carbonate (MCC or SRCC) may be prepared by providing a GCC or PCC in form of an aqueous suspension, and adding an acid to said suspension. Suitable acids are, for example, sulphuric acid, hydrochloric acid, phosphoric acid, citric acid, oxalic acid, or a mixture thereof. In a next step, the calcium carbonate is treated with gaseous carbon dioxide. If a strong acid such as sulphuric acid or hydrochloric acid is used for the acid treatment step, the carbon dioxide will form automatically in situ. Alternatively or additionally, the carbon dioxide can be supplied from an external source. Surface-reacted calcium carbonates are described, for example, in US20120031576 A1, WO2009074492 A1, EP2264109 A1, EP2070991 A1, EP2264108 A1, WO0039222 A1, WO2004083316 A1 or WO2005121257 A2.

The natural ground calcium carbonate and/or synthetic precipitated calcium carbonate and/or surface-reacted calcium carbonate may additionally be surface treated or may comprise a dispersing agent well known to the skilled person. For example, the dispersing agent may be an acrylate-based dispersing agent.

If the aqueous preparation comprises at least one inorganic particulate material, the at least one inorganic particulate material may have a particle size distribution as conventionally employed for the material(s) involved in the type of product to be produced. In general, 90 % of the particles will have an esd (equivalent spherical diameter as measured by the well-known technique of sedimentation using Sedigraph 5120 series, Micromeritics) of less than 5 µm. Coarse inorganic particulate materials may have a particle esd generally (i.e., at least 90 wt.-%) in the range of 1 to 5 µm. Fine inorganic particulate materials may have a particle esd generally less than 2 µm, e.g. 50.0 to 99.0 wt.-% less than 2 µm and preferably 60.0 to 90.0 wt.-% less than 2 µm. It is preferred that the at least one inorganic particulate material in the aqueous preparation has a weight median particle size dso value of from 0.1 to 5 µm, preferably from 0.2 to 2 µm and most preferably from 0.35 to 1 µm, for example 0.7 µm as measured using a SedigraphTM 5120 of Micromeritics Instrument Corporation.

For keeping such inorganic particulate materials dispersed in an aqueous preparation and thus ensuring that the viscosity of the preparation remains substantially the same over time, additives such as dispersing agents can be used. A suitable dispersing agent according to the present invention is preferably a homo or copolymer made of monomers and/or co-monomers selected from the group consisting of acrylic acid, methacrylic acid, itaconic acid, crotonic acid, fumaric acid, maleic anhydride acid, isocrotonic acid, aconitic acid (cis or trans), mesaconic acid, sinapinic acid, undecylenic acid, angelic acid, canellic acid, hydroxyacrylic acid, acrolein, acrylamide, acrylonitrile, dimethylaminoethyl methacrylate, vinylpyrrolidone, styrene, the esters of acrylic and methacrylic acids and mixtures thereof, wherein salts of poly(acrylic acid) and/or poly (methacrylic acid) are preferred as dispersing agent.

Additionally or alternatively, the aqueous preparation comprises at least one organic particulate material. For example, the at least one organic material is selected from the group comprising carbohydrates such as starch, sugar, cellulose, modified cellulose and cellulose based pulp, glycerol, hydrocarbons, water-soluble polymers such as polyacrylates, and mixtures thereof.

In one embodiment of the present invention, the aqueous preparation comprises at least one inorganic particulate material, preferably being selected from the group comprising natural ground calcium carbonate, natural and/or synthetic precipitated calcium carbonate, surface-reacted calcium carbonate, dolomite, calcium sulphate, kaolin, clay, barite, talcum, quartz, mica, gypsum, aluminium hydroxide, aluminium silicate, titanium dioxide, magnesite, hydromagnesite, hydroxylapatite, perlite, sepiolite, brucite and mixtures thereof, and most preferably the at least one inorganic particulate material comprises natural ground calcium carbonate and/or synthetic precipitated calcium carbonate.

Thus, the aqueous preparation is preferably an aqueous suspension (or slurry).

It is appreciated that the solids content of the aqueous preparation can be up to 85.0 wt.-%. For example, the solids content of the aqueous preparation is from 05.0 to 82.0 wt.-%, preferably from 0.5.0 to 80.0 wt.-%, and most preferably from 0.5 to 60.0 wt.-%, based on the total weight of the aqueous preparation.

The total solids content in the meaning of the present application corresponds to the residual weight of the aqueous preparation after drying for 3 h at 105°C as measured in a sample of at least 3 to 5 g.

It is to be noted that the aqueous preparation may comprise buffer components differing from the first and second buffer components of the present buffer composition. Thus, the aqueous preparation may comprise buffer components selected from the group comprising amine-based buffer components, monoalcohol-based buffer components, primary alkanol amine-based buffer components, hydroxide-based buffer components and mixtures thereof. For example, the aqueous preparation may comprise buffer components selected from the group comprising ammonia, methylamine, dimethylamine, trimethylamine, diethylamine, propylamine, butylamine, hexylamine, amino-2-methylpropanol (AMP), monoethanolamine (MEA), monoisopropanolamine (MIPA), triethylenetetramine (TETA), calcium hydroxide and mixtures thereof.

The pH of the aqueous preparation can vary in a broad range and is preferably in a pH range typically observed for such aqueous preparations. It is thus appreciated that the aqueous preparation preferably has a pH value of from 2 to 12. For example, the aqueous preparation of step a) has a pH value of from 6 to 11.5 and more preferably from 7 to 10.5.

Typically, the aqueous preparation has a viscosity being preferably in the range from 50 to 2 000 mPa·s and preferably from 80 to 800 mPa·s, as measured with a Brookfield DV-II Viscometer at a speed of 100 rpm and equipped with a LV-3 spindle.

The aqueous preparations according to the invention can be produced by methods known in the art, by for example, dispersing, suspending or slurring water-insoluble solids, preferably inorganic particulate materials with, if appropriate, addition of a dispersing agent and, if appropriate, further additives in water.

As already mentioned above, the buffer composition is capable of maintaining the pH of an article being an aqueous preparation equal to or below 12, preferably from 4 to 12, more preferably from 6 to 11.5 and most preferably from 8.5 to 10.5.

Thus, it is appreciated that the article being an aqueous preparation has a pH value of equal to or below 12, preferably from 4 to 12, more preferably from 6 to 11.5 and most preferably from 8.5 to 10.5.

The present invention also refers to a process for buffering an aqueous preparation, said process comprises the steps of
a) providing an aqueous preparation, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation,
b) providing a buffer composition as defined herein, and
c) contacting and mixing the aqueous preparation of step a) one or more times with the buffer composition of step b) for obtaining the buffered aqueous preparation.

With regard to the definition of the aqueous preparation, the buffer composition and preferred embodiments thereof, reference is made to the statements provided above when discussing the technical details of the aqueous preparation and the buffer composition of the present invention.

According to step c) of the process of the present invention, the aqueous preparation of step a) is contacted and mixed with the buffer composition of step b).

It is thus appreciated that the buffer composition is preferably capable of maintaining the pH of an aqueous preparation or a solid article equal to or below 12, preferably from 4 to 12, more preferably from 6 to 11.5 and most preferably from 8.5 to 10.5, when the buffer composition is present. This prevents the increase or decrease of the pH in the buffered aqueous preparation.

In general, the aqueous preparation of step a) and the buffer composition of step b) can be brought into contact by any conventional means known to the skilled person.

It is appreciated that step c) is preferably carried out by adding the buffer composition of step b) to the aqueous preparation of step a).

Preferably, the step c) is carried out in that the buffer composition is added to the aqueous preparation under mixing. A sufficient mixing may be achieved by shaking the aqueous preparation or by agitation, which may provide a more thorough mixing. In one embodiment of the present invention, step c) is carried out under agitation to ensure a thorough mixing of the aqueous preparation and the buffer composition. Such agitation can be carried out continuously or discontinuously.

In one embodiment, step c) is carried out in that the buffer composition is added to the aqueous preparation in an amount such that the first and the second buffer components are present in the aqueous preparation in a total amount of at least 100 ppm, e.g. from 100 to 27 000 ppm, preferably at least 250 ppm, e.g. from 250 to 25 000 ppm, more preferably at least 500 ppm, e.g. from 500 to 20 000 ppm, still more preferably at least 600 ppm, e.g. from 600 to 15 000 ppm, even more preferably at least 750 ppm, e.g. from 750 to 10 000 ppm, and most preferably from 750 to 5 000 ppm, calculated relative to the total weight of the aqueous preparation.

It is appreciated that the amount of each of the first and the second buffer components and the optional at least one water soluble or water dispersable source of sodium ions can vary in a great range in the aqueous preparation.

It is to be noted that the aforementioned figures reflect the amount of the buffer composition being added via the first and the second buffer components and the optional at least one water soluble or water dispersable source of sodium ions to the aqueous preparation and do not cover any dissolved magnesium ions and/or zinc ions, alkali carbonate and/or alkali bicarbonate ions and sodium ions which may naturally be present in the aqueous preparation.

It is appreciated that the single components of the buffer composition can be added to the aqueous preparation as a pre-mixed composition or in form of the single components.

In one embodiment, the pre-mixed composition or the single components of the buffer composition can be added to the aqueous preparation in dry form, such as in form of a powder, granules, pellets or tablets, or in form of a solution or suspension or dispersion. Preferably, the pre-mixed composition or the single components of the buffer composition is/are added to the aqueous preparation in form of a powder.

The amount of the first and the second buffer components and the optional at least one water soluble or water dispersable source of sodium ions added to the aqueous preparation can be individually adjusted depending on the aqueous preparation. In particular, the amount of the buffer composition and the single components therein depends on the nature and the occurrence of the first and the second buffer components and the optional at least one water soluble or water dispersable source of sodium ions to be used in the aqueous preparation. The optimum amount to be employed within the defined ranges can be determined by preliminary tests and test series on a laboratory scale and by supplementary operational tests.

It is appreciated that step c) can be repeated one or more times.

The buffer composition can be added in one or several portions to the aqueous preparation. If the buffer composition is added in several portions, the buffer composition can be added in about equal portions or unequal portions to the aqueous preparation.

The aqueous preparation obtained in step c) preferably has solids content corresponding to the solids content of the aqueous preparation provided in step a). It is thus appreciated that the aqueous preparation obtained in step c) preferably has solids content of up to 85.0 wt.-%, based on the total weight of the aqueous preparation obtained in step c). For example, the solids content of the aqueous preparation obtained in step c) is from 0.5 to 82.0 wt.-%, preferably from 0.5 to 80.0 wt.-%, and most preferably from 0.5 to 60.0 wt.-%, based on the total weight of the aqueous preparation obtained in step c).

In particular, the pH of the aqueous preparation obtained in step c) is preferably equal to or below 12, preferably from 4 to 12, more preferably from 6 to 11.5 and most preferably from 8.5 to 10.5.

Typically, the aqueous preparation obtained in step c) has a viscosity being preferably in the range from 50 to 2 000 mPa·s and preferably from 80 to 800 mPa·s, as measured with a Brookfield DV-II Viscometer at a speed of 100 rpm and equipped with a LV-3 spindle.

The aqueous preparation is preferably obtainable by the process of the instant invention, i.e. the process for buffering an aqueous preparation as defined above.

A further aspect of the present thus refers to an aqueous preparation, preferably a buffered aqueous preparation, obtainable by a process for buffering an aqueous preparation as defined herein.

As already mentioned above, the buffer composition of the present invention can be also used for maintaining the pH of an article being a solid article equal to or below 12. Thus, the article can be any kind of solid article in need of a pH stabilization.

In one embodiment, the solid article can be a coating, paint film, lacquer or coating, paper coating, paper, paperboard, adhesive, sealant, pigment, fiber, plaster, plaster-spray, plasterboard, binder, thickener and/or concrete.

In one embodiment, the solid article comprises the first and the second buffer components of the buffer composition in a total amount of at least 100 ppm, e.g. from 100 to 27 000 ppm, preferably at least 250 ppm, e.g. from 250 to 25 000 ppm, more preferably at least 500 ppm, e.g. from 500 to 20 000 ppm, still more preferably at least 600 ppm, e.g. from 600 to 15 000 ppm, even more preferably at least 750 ppm, e.g. from 750 to 10 000 ppm, and most preferably from 750 to 5 000 ppm, calculated relative to the total weight of the solid article.

Thus, it is preferred that the aqueous preparation comprises the buffer composition such that
a) the first buffer component being at least one water soluble or water dispersable source of magnesium ions and/or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate and/or bicarbonate ions] is from 10 000:1 to 1:10 000.

The first buffer component can be at least one water soluble or water dispersable source of magnesium ions and zinc ions. In this case, the solid article comprises the buffer composition such that
a) the first buffer component being at least one water soluble or water dispersable source of magnesium ions and zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and Zn ions / carbonate and/or bicarbonate ions] is from 10 000:1 to 1:10 000.

The first buffer component can be at least one water soluble or water dispersable source of magnesium ions or zinc ions. In this case, the solid article comprises the buffer composition such that
a) the first buffer component being at least one water soluble or water dispersable source of magnesium ions or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg or Zn ions / carbonate and/or bicarbonate ions] is from 10 000:1 to 1:10 000.

The second buffer component can be at least one alkali carbonate and at least one alkali bicarbonate. In this case, the solid article comprises the buffer composition such that
a) the first buffer component being at least one water soluble or water dispersable source of magnesium ions and/or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate and bicarbonate ions] is from 10 000:1 to 1:10 000.

The second buffer component can be at least one alkali carbonate or at least one alkali bicarbonate. In this case, the solid article comprises the buffer composition such that
a) the first buffer component being at least one water soluble or water dispersable source of magnesium ions and/or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate or bicarbonate ions] is from 10 000:1 to 1:10 000.

The first buffer component can be at least one water soluble or water dispersable source of magnesium ions and zinc ions and the second buffer component can be at least one alkali carbonate and at least one alkali bicarbonate. In this case, the solid article comprises the buffer composition such that
a) the first buffer component being at least one water soluble or water dispersable source of magnesium ions and zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and Zn ions / carbonate and bicarbonate ions] is from 10 000:1 to 1:10 000.

The first buffer component can be at least one water soluble or water dispersable source of magnesium ions or zinc ions and the second buffer component can be at least one alkali carbonate or at least one alkali bicarbonate. In this case, the solid article comprises the buffer composition such that
a) the first buffer component being at least one water soluble or water dispersable source of magnesium ions or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg or Zn ions / carbonate or bicarbonate ions] is from 10 000:1 to 1:10 000.

For example, the solid article comprises the buffer composition such that
a) the first buffer component being at least one water soluble or water dispersable source of zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Zn ions / carbonate ions] is from 10 000:1 to 1:10 000.

If the buffer composition further comprises at least one water soluble or water dispersable source of sodium ions, the solid article comprises the buffer composition such that
a) the first buffer component being at least one water soluble or water dispersable source of magnesium ions and/or zinc ions is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate is present in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions is present in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate and/or bicarbonate ions] is from 10 000:1 to 1:10 000.

In case the first buffer component comprises at least one water soluble or water dispersable source of magnesium ions and zinc ions, the solid article comprises
a) the first buffer component being at least one water soluble or water dispersable source of magnesium ions and zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and Zn ions / carbonate and/or bicarbonate ions] is from 10 000:1 to 1:10 000.

In case the first buffer component comprises at least one water soluble or water dispersable source of magnesium ions or zinc ions, the solid article comprises
a) the first buffer component being at least one water soluble or water dispersable source of magnesium ions or zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and/or at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg or Zn ions / carbonate and/or bicarbonate ions] is from 10 000:1 to 1:10 000.

In case the second buffer component comprises at least one alkali carbonate and at least one alkali bicarbonate, the solid article comprises
a) the first buffer component being at least one water soluble or water dispersable source of magnesium ions and/or zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate and bicarbonate ions] is from 10 000:1 to 1:10 000.

In case the second buffer component comprises at least one alkali carbonate or at least one alkali bicarbonate, the solid article comprises
a) the first buffer component being at least one water soluble or water dispersable source of magnesium ions and/or zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate or at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and/or Zn ions / carbonate or bicarbonate ions] is from 10 000:1 to 1:10 000.

In case the first buffer component comprises at least one water soluble or water dispersable source of magnesium ions and zinc ions and the second buffer component comprises at least one alkali carbonate and at least one alkali bicarbonate, the solid article comprises
a) the first buffer component being at least one water soluble or water dispersable source of magnesium ions and zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate and at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg and Zn ions / carbonate and bicarbonate ions] is from 10 000:1 to 1:10 000.

In case the first buffer component comprises at least one water soluble or water dispersable source of magnesium ions or zinc ions and the second buffer component comprises at least one alkali carbonate or at least one alkali bicarbonate, the solid article comprises
a) the first buffer component being at least one water soluble or water dispersable source of magnesium ions or zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate or at least one alkali bicarbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Mg or Zn ions / carbonate or bicarbonate ions] is from 10 000:1 to 1:10 000.

For example, the solid article comprises
a) the first buffer component being at least one water soluble or water dispersable source of zinc ions in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
b) the second buffer component being at least one alkali carbonate in an amount of at least 10 ppm, e.g. from 10 to 27 000 ppm, more preferably at least 25 ppm, e.g. from 25 to 25 000 ppm, even more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article, and
c) the at least one water soluble or water dispersable source of sodium ions in an amount of at least 10 ppm, e.g. from 10 to 27 000 ppm, preferably at least 25 ppm, e.g. from 25 to 25 000 ppm, more preferably at least 50 ppm, e.g. from 50 to 20 000 ppm, still more preferably at least 60 ppm, e.g. from 60 to 15 000 ppm, even more preferably at least 75 ppm, e.g. from 75 to 10 000 ppm, and most preferably from 75 to 5 000 ppm, calculated relative to the total weight of the solid article,
wherein the molar ratio of the first buffer component to the second buffer component [Zn ions / carbonate ions] is from 10 000:1 to 1:10 000.

It is appreciated that the amount of each of the first and the second buffer components and the optional at least one water soluble or water dispersable source of sodium ions can vary in a great range in the solid article.

It is to be noted that the aforementioned figures reflect the amount of the buffer composition being added via the first and the second buffer components and the optional at least one water soluble or water dispersable source of sodium ions to the solid article and do not cover any dissolved magnesium ions and/or zinc ions, alkali carbonate and sodium ions which may naturally be present in the solid article.

It is appreciated that the solid article is preferably obtained by drying, solidifying or hardening the aqueous preparation described above, i.e. a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation.

Thus, the solid article preferably comprises at least one inorganic particulate material.

For example, the at least one inorganic particulate material is selected from the group comprising natural ground calcium carbonate, natural and/or synthetic precipitated calcium carbonate, surface-reacted calcium carbonate, dolomite, calcium sulphate, kaolin, clay, barite, talcum, quartz, mica, gypsum, aluminium hydroxide, aluminium silicate, titanium dioxide, magnesite, hydromagnesite, hydroxylapatite, perlite, sepiolite, brucite and mixtures thereof.

In one embodiment of the present invention, the at least one inorganic particulate material comprises natural ground calcium carbonate and/or synthetic precipitated calcium carbonate and/or surface-reacted calcium carbonate. Preferably, the at least one inorganic particulate material comprises natural ground calcium carbonate and/or synthetic precipitated calcium carbonate.

It is to be noted that hydromagnesite and brucite are either present as the first buffer component of the buffer composition or the at least one inorganic particulate material of the solid article. That is to say, if the solid article comprises hydromagnesite and/or brucite as the at least one inorganic particulate material, hydromagnesite and/or brucite is/are not included as the first buffer component in the buffer composition.

The natural ground calcium carbonate and/or synthetic precipitated calcium carbonate and/or surface-reacted calcium carbonate may additionally be surface treated or may comprise a dispersing agent well known to the skilled person. For example, the dispersing agent may be an acrylate-based dispersing agent.

If the solid article comprises at least one inorganic particulate material, the at least one inorganic particulate material may have a particle size distribution as conventionally employed for the material(s) involved in the type of product to be produced. In general, 90 % of the particles will have an esd (equivalent spherical diameter as measured by the well-known technique of sedimentation using Sedigraph 5100 series, Micromeritics) of less than 5 µm. Coarse inorganic particulate materials may have a particle esd generally (i.e., at least 90 wt.-%) in the range of 1 to 5 µm. Fine inorganic particulate materials may have a particle esd generally less than 2 µm, e.g. 50.0 to 99.0 wt.-% less than 2 µm and preferably 60.0 to 90.0 wt.-% less than 2 µm. It is preferred that the at least one inorganic particulate material in the aqueous preparation has a weight median particle size d₅₀ value of from 0.1 to 5 µm, preferably from 0.2 to 2 µm and most preferably from 0.35 to 1 µm, for example 0.7 µm as measured using a SedigraphTM 5100 of Micromeritics Instrument Corporation.

The solid article may further comprise dispersing agents. A suitable dispersing agent according to the present invention is preferably a homo or copolymer made of monomers and/or co-monomers selected from the group consisting of acrylic acid, methacrylic acid, itaconic acid, crotonic acid, fumaric acid, maleic anhydride acid, isocrotonic acid, aconitic acid (cis or trans), mesaconic acid, sinapinic acid, undecylenic acid, angelic acid, canellic acid, hydroxyacrylic acid, acrolein, acrylamide, acrylonitrile, dimethylaminoethyl methacrylate, vinylpyrrolidone, styrene, the esters of acrylic and methacrylic acids and mixtures thereof, wherein salts of poly(acrylic acid) and/or poly (methacrylic acid) are preferred as dispersing agent.

Additionally or alternatively, the solid article comprises at least one organic particulate material. For example, the at least one organic material is selected from the group comprising carbohydrates such as starch, sugar, cellulose, modified cellulose and cellulose based pulp, glycerol, hydrocarbons, water-soluble polymers such as polyacrylates, and mixtures thereof.

It is to be noted that the solid article may comprise buffer components differing from the first and second buffer components of the present buffer composition. Thus, the solid article may comprise buffer components selected from the group comprising amine-based buffer components, monoalcohol-based buffer components, primary alkanol amine-based buffer components, hydroxide-based buffer components and mixtures thereof. For example, the solid article may comprise buffer components selected from the group comprising ammonia, methylamine, dimethylamine, trimethylamine, diethylamine, propylamine, butylamine, hexylamine, amino-2-methylpropanol (AMP), monoethanolamine (MEA), monoisopropanolamine (MIPA), triethylenetetramine (TETA), calcium hydroxide and mixtures thereof. Typically, the buffer components differing from the first and second buffer components of the present buffer composition are included before the present buffer composition is added.

The solid articles according to the invention can be produced by methods known in the art by for example drying, solidifying or hardening the aqueous preparation described above, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation.

As already mentioned above, the buffer composition is capable of maintaining the pH of an article being a solid article equal to or below 12, preferably from 4 to 12, more preferably from 6 to 11.5 and most preferably from 8.5 to 10.5.

Thus, it is appreciated that the article being a solid article has a pH value of equal to or below 12, preferably from 4 to 12, more preferably from 6 to 11.5 and most preferably from 8.5 to 10.5.

The present invention also refers to a process for buffering a solid article, said process comprises the steps of
a) providing a solid article, preferably a coating, paint film, lacquer or coating, paper coating, paper, paperboard, adhesive, sealant, pigment, fiber, plaster, plaster-spray, plasterboard, binder, thickener and/or concrete comprising the buffer composition as defined herein, and
b) moisten the surface of the solid article of step a) for obtaining the buffered solid article.

With regard to the definition of the solid article, the buffer composition and preferred embodiments thereof, reference is made to the statements provided above when discussing the technical details of the solid article and the buffer composition of the present invention.

According to step b) of the process of the present invention, the surface of the solid article of step a) is moisten for obtaining the buffered solid article. That is to say, the moistening of the surface of the solid article results in a reactivation of the buffering capacity of the buffer composition of the present invention. Furthermore, it is to be noted that the solid article, without being moistened, does not provide a buffering capacity.

It is thus appreciated that the buffer composition is preferably capable of maintaining the pH of the moisten solid article equal to or below 12, preferably from 4 to 12, more preferably from 6 to 11.5 and most preferably from 8.5 to 10.5, when the buffer composition is present. This prevents the increase or decrease of the pH on the surface of the buffered solid article.

In general, the surface of the solid article of step a) can be moisten by any conventional means known to the skilled person.

It is appreciated that step b) is preferably carried out by contacting the surface of the solid article of step a) with water by e.g. wetting, spraying, condensing, brushing, dipping, immersing etc., or if the surface of the solid article of step a) is exposed to humidity or rain.

The amount of the first and the second buffer components and the optional at least one water soluble or water dispersable source of sodium ions added to the solid article can be individually adjusted depending on the solid article. In particular, the amount of the buffer composition and the single components therein depends on the nature and the occurrence of the first and the second buffer components and the optional at least one water soluble or water dispersable source of sodium ions to be used in the solid article. The optimum amount to be employed within the defined ranges can be determined by preliminary tests and test series on a laboratory scale and by supplementary operational tests.

It is appreciated that step b) can be repeated one or more times.

The pH of the buffered solid article obtained in step b) is equal to or below 12, preferably from 4 to 12, more preferably from 6 to 11.5 and most preferably from 8.5 to 10.5.

The buffered solid article is preferably obtainable by the process of the instant invention, i.e. the process for buffering a solid article as defined above.

In view of the goods results obtained, the present invention refers in a further aspect to the use of the buffer composition as defined herein for maintaining the pH of an article, preferably an aqueous preparation or a solid article, equal to or below 12, preferably from 4 to 12, more preferably from 6 to 11.5 and most preferably from 8.5 to 10.5.

With regard to the definition of the article, the buffer composition and preferred embodiments thereof, reference is made to the statements provided above when discussing the technical details of the article and the buffer composition of the present invention.

The scope and interest of the present invention will be better understood based on the following examples which are intended to illustrate certain embodiments of the present invention and are non-limitative.

### Examples

### (A) Analytical methods

### Particle size distributions

The particle size of surface-reacted calcium carbonate (SRCC) herein is described as volume-based particle size distribution dx. The volume-based median particle size *d*₅₀ and the volume-based top cut *d*₉₈ were measured using a Malvern Mastersizer 2000
Laser Diffraction System (Malvern Instruments Plc., Great Britain). The raw data obtained by the measurement was analyzed using the Mie theory, with a particle refractive index of 1.57 and an absorption index of 0.005. The methods and instruments are known to the skilled person and are commonly used to determine particle size distributions of fillers and pigments.

The particle size of particulate materials other than surface-reacted calcium carbonate (e.g. ground natural calcium carbonate, GNCC) is described herein as weight-based particle size distribution dx. The weight determined median particle size *d*₅₀ and top cut *d*₉₈ were measured by the sedimentation method, which is an analysis of sedimentation behaviour in a gravimetric field. The measurement was made with a Sedigraph^{™} 5120 of Micromeritics Instrument Corporation, USA. The method and the instrument are known to the skilled person and are commonly used to determine particle size distributions of fillers and pigments. The measurement was carried out in an aqueous solution of 0.1 wt% Na₄P₂O₇. The samples were dispersed using a high speed stirrer and sonicated.

### BET specific surface area (SSA)

Throughout the present document, the specific surface area (in m²/g) was determined using the BET method (using nitrogen as adsorbing gas), which is well known to the skilled man (ISO 9277:2010). The total surface area (in m2) of the filler material was then obtained by multiplication of the specific surface area and the mass (in g) of the corresponding sample.

### Brookfield viscosity

The Brookfield viscosity was measured by a Brookfield DV-III Ultra viscometer at 24 °C ± 3 °C at 100 rpm using an appropriate spindle of the Brookfield RV-spindle set and is specified in mPa·s. Once the spindle has been inserted into the sample, the measurement was started with a constant rotating speed of 100 rpm. The reported Brookfield viscosity values were the values displayed 60 seconds after the start of the measurement. Based on his technical knowledge, the skilled person will select a spindle from the Brookfield RV-spindle set which is suitable for the viscosity range to be measured. For example, for a viscosity range between 100 and 400 mPa·s the spindle number 2 may be used, for a viscosity range between 400 and 1 600 mPa·s the spindle number 4 may be used, for a viscosity range between 800 and 3 200 mPa·s the spindle number 5 may be used, for a viscosity range between 1 000 and 2 000 000 mPa·s the spindle number 6 may be used, and for a viscosity range between 4 000 and 8 000 000 mPa·s the spindle number 7 may be used.

### Solids content

The suspension solids content (also known as "dry weight") was determined using a Moisture Analyser MJ33 (Mettler-Toledo, Switzerland), with the following settings: drying temperature of 150 °C, automatic switch off if the mass does not change more than 1 mg over a period of 30 s, standard drying of 5 g of suspension.

### pH measurement

Any pH value was measured at 25°C (+/- 1°C) using a Mettler Toledo Seven Easy pH meter and a Mettler Toledo InLab Expert Pro pH electrode. A three point calibration of the instrument was first made using commercially available buffer solutions having pH values of 4, 7 and 10 at 25°C (from Aldrich). The reported pH values were the endpoint values detected by the instrument (signal differs by less than 0.1 mV from the average over the last 6 seconds).

### (B) Examples

The following examples are not to be construed to limit the scope of the claims in any matter whatsoever.

### Materials

### a. White base paint

A white base paint as shown in Table 1 was used for the challenging tests.

**Table 1 -White base paint:**

| | |
|---|---|
| Water deionized | 32.2 |
| Calgon N New | 0.1 |
| Bermocoll Prime 3500 | 0.5 |
| Sodium hydroxide, 10 % | 0.1 |
| Byk 038 | 0.3 |
| Coapur2025 | 0.4 |
| Mergal 723 K | 0.2 |
| Ecodis P 50 | 0.3 |
| | |
| Titanium dioxide Tiona^{®}595 | 6.0 |
| Finntalc M20SL-AW | 5.0 |
| Omyacarb extra-CL | 13.0 |
| Omyacarb 5-GU | 23.0 |
| Omya-Calcimatt-A V | 7.0 |
| | |
| Byk 012 | 0.4 |
| Mowilith LDM 1871, 53 % | 11.5 |
| | |
| **Total** | **100.0** |
| | |
| *Solids content [wt%]* | *61.6* |
| *pH* | *8.3 - 8.8* |
| *Viscosity* | *170 mPas* |

The components used for the white base paint and their function are known to the skilled person and listed in Table 2 hereto below.

**Table 2 - Materials for the white base paint:**

| **White base paint** | **Producer** | **Chemical basis** | **Function** |
|---|---|---|---|
| Water | In house, deionized | H₂O | Solvent |
| Calgon N new | BK Giulini Chemie | Sodium polyphosphate | Wetting and dispersing agent |
| Bermocoll Prime 3500 | AkzoNobel Corp. | Methyl ethyl hydroxyethyl cellulose | Rheology modifier |
| Sodium hydroxide, 10 % | Various | NaOH solution | pH regulator |
| BYK 038 | Byk Chemie | Mineral oil basis | Defoamer |
| Coapur2025 | Coatex SA | Polyurethane | Rheology modifier |
| Mergal 723K | Troy Chemie GmbH | Benzisothiazolone basis, without formaldehyde | Biocide |
| ECODIS P 50 | Coatex SA | Polyacrylate sodium salt | Wetting and dispersing agent |
| | | | |
| Titanium dioxide Tiona ^{®} 595 | Tronox | Rutile Titanium dioxide | White pigment |
| Finntalc M20SL-AW | Elementis Global | Talc (Hydrated Magnesium silicate: Mg₃Si₄O₁₀(OH)₂) | Functional white extender |
| Omyacarb extra-CL ^{#1} | Omya | Calcium carbonate | Functional white extender |
| Omyacarb 5-GU ^{#2} | Omya | Calcium carbonate | Functional white extender |
| Omya-Calcimatt - AV ^{#3} | Omya | Calcium carbonate | Functional matting agent |
| | | | |
| Byk 012 | Byk Chemie | Polymer and hydrophilic particle based material | Defoamer |
| Mowilith LDM 1871, 53% | Celanese Emulsion Polymers | Aqueous copolymer dispersion based on vinyl acetate and ethylene | Binder (copolymer) |

| | | | |
|---|---|---|---|
| ^{#1} The extender relates to a dry ground calcium carbonate (marble from Italy) having a median diameter (*d*₅₀) of 0.8 µm and a top cut (*d*₉₈) of 5 µm which is commercially available. ^{#2} The extender relates to a dry ground calcium carbonate (marble from Italy) having a median diameter (*d*₅₀) of 4.5-6.5 µm, a sieve residue > 100 µm of ≤ 30 ppm, and sieve residue > 45 µm of ≤ 0.1 % (ISO 787/7), which is commercially available as Omyacarb 5 - GU from Omya International AG. ^{#3} The matting agent relates to a dry ground calcium carbonate (marble from Italy) having a median diameter (*d*₅₀) of 20 µm, a sieve residue > 60 µm of ≤ 0.8 % and a sieve residue > 45 µm of ≤ 2% (ISO 787 /7), which is commercially available as Omya Calcimatt AV from Omya International AG. | | | |

### b. Preparation of the paint for: Challenge Test No.1.

Fresh white base paint was prepared by combining all substances according to the amounts set out in Table 1 and aliquoted into 50 g samples. Triplicates for each additive (0.3%), i.e. for ZnO, NaOH 10M, AMP, Ammonia, Li₂CO₃, and BC1 (Buffer Composition 1 ZnO:Li₂CO₃ 1:1 by weight) and untreated white base paint as negative control, were prepared according to the following table 3 and the pH was measured directly after addition of the test substances at room temperature (RT), [t=0]. Thereafter 1.25 ml of 0.1M HCL was added mixed and after 3 weeks [t=3], the pH was measured again.

**Table 3 - Tests prepared**

| **Test** | **Temp.** | **Additive 0.3%** | **[mg] or [µl]** | **pH [t=0]** | **Average** | **STDV** | **pH [t=3]** | **Average** | **STDV** |
|---|---|---|---|---|---|---|---|---|---|
| 1a | RT | neg. | - | 8.21 | 8.097 | 0.121 | 7.91 | 7.927 | 0.029 |
| 1b | RT | neg. | - | 7.97 | | | 7.91 | | |
| 1c | RT | neg. | - | 8.11 | | | 7.96 | | |
| 2a | RT | ZnO | 150 mg | 8.75 | 8.743 | 0.071 | 9.02 | 8.957 | 0.071 |
| 2b | RT | ZnO | 150 mg | 8.78 | | | 8.97 | | |
| 2c | RT | ZnO | 150 mg | 8.70 | | | 8.88 | | |
| 3a | RT | NaOH (10 M) | 375 µl | 12.21 | 12.237 | 0.046 | 10.56 | 10.553 | 0.021 |
| 3b | RT | NaOH (10 M) | 375 µl | 12.29 | | | 10.53 | | |
| 3c | RT | NaOH (10 M) | 375 µl | 12.21 | | | 10.57 | | |
| 4a | RT | AMP | 150 mg | 10.09 | 10.130 | 0.069 | 9.70 | 9.747 | 0.081 |
| 4b | RT | AMP | 150 mg | 10.21 | | | 9.84 | | |
| 4c | RT | AMP | 150 mg | 10.09 | | | 9.70 | | |
| 5a | RT | Li₂CO₃ | 150 mg | 9.89 | 9.883 | 0.100 | 9.63 | 9.707 | 0.075 |
| 5b | RT | Li₂CO₃ | 150 mg | 9.98 | | | 9.71 | | |
| 5c | RT | Li₂CO₃ | 150 mg | 9.78 | | | 9.78 | | |
| 6a | RT | NH₃ | 600 µl | 10.42 | 10.420 | 0.000 | 10.22 | 10.223 | 0.015 |
| 6b | RT | NH₃ | 600 µl | 10.42 | | | 10.24 | | |
| 6c | RT | NH₃ | 600 µl | 10.42 | | | 10.21 | | |
| 7a | RT | BC1 | 150 mg | 9.60 | 9.610 | 0.075 | 9.62 | 9.587 | 0.031 |
| 7b | RT | BC1 | 150 mg | 9.54 | | | 9.56 | | |
| 7c | RT | BC1 | 150 mg | 9.69 | | | 9.58 | | |

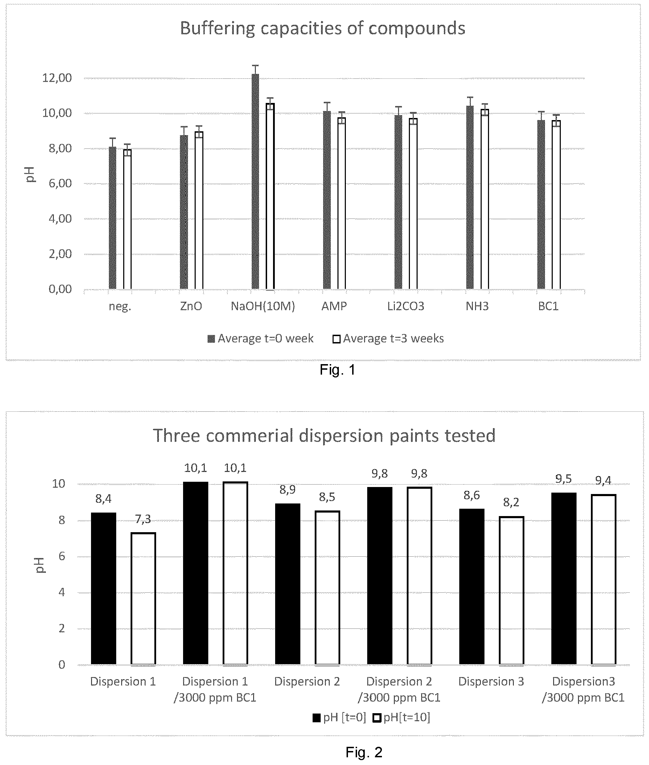

The results are also shown in Fig. 1

As can be gathered from Table 3 and Fig. 1, the buffering capacities of the different substances show that pH changes occur over the course of three weeks.

### c. Preparation of dispersion paints for: Challenge Test No. 2

From a Do-it-Yourself Store, three commercially available indoor dispersion paints were tested in double. For each paint two 50 g dispersion paint samples were prepared. One was provided with 3000 ppm of BC1 composition, whereas the untreated paint was used as control. The pH of the samples was measured at t=0, i.e after the addition of 3 000 ppm of BC1 (Buffer Composition 1 ZnO:Li₂CO₃ 1:1 by weight) and 10 weeks (t=10) after the addition of 3 000 ppm of BC1 (Buffer Composition 1 ZnO:Li₂CO₃ 1:1 by weight). The results are shown in Fig. 2 below.

Fig. 2 shows that commercial available dispersion paints can be adjusted to a certain pH value and that the adjusted pH value can be maintained over a period of at least 10 week, whereas the non-adjusted commercial dispersion paints show a pH decrease of at least 0.4 pH units over the same time range. BC1 (Buffer Composition 1 ZnO:Li₂CO₃ 1:1 by weight) thus effectively maintains the pH at the initial adjusted pH level of at least 10 weeks.

### (C) Computer simulations and Experimental Verification

Better insight into the equilibriums of a calcium carbonate slurry was achieved by numerical model calculations in this study. The computer software PHREEQC in version 3.6 has been used to model the equilibrium chemistry of aqueous solutions interacting with minerals and gases. It solves a reduced set of simultaneous non-linear equations that define equilibrium among aqueous speciation and reactions by a modified Newton-Raphson calculation. The database MINTEQA2 provided with the software has been used in this study with some additions to include all equilibrium data relevant for this study. This includes equilibrium data forCa-/Zn-lactate complexes (Maia et al. 2016, Krezel&Maret 2016), Ca-/Zn-lactate and citrate solids (Vavrusova et al. 2013, Apelblat et al. 2005, Vavrusova & Skibsted 2016, Christrie et al. 1991) obtained directly from the literature. Equilibrium solubility data for Ca-Al phases have been transferred from the Lawrence Livermoore National Laboratory database included in PHREEQC. Equilibrium solubility data for the lithium carbonate mineral Zabuleyite (Li₂CO₃) was calculated from thermodynamic data of Zabuleyite (Anderson et al. 2001) and aqueous species thermodynamic data from SUPCRTBL database (Zimmer et al. 2016). The equilibrium data for aqueous species of 2-Amino-2-methylpropanol has been added from Littel et al. 1990. The extension of the database also included the addition of equilibrium dataset for nitrogen and ammonia from the WATEQ4F database included in PHREEQC for explicitly simulating a gas phase.

High solid suspensions of different inorganic particulate materials have been simulated by an aqueous suspension of 60 wt.-% of the particulate material and 40 wt.-% of water as the base composition at the starting point of the simulation. To this suspension BC1 (Buffer Composition 1 ZnO:Li₂CO₃ 1:1 by weight)has been added in equilibrium with defined maximum amounts of pure phases to investigate their influence on the pH of the aqueous suspension. The system is in addition also influenced by gaseous components, which have been taken into account using four different approaches. The most simple approach does not allow any removal of any component from the aqueous-solid system even if equilibrium is supersaturated. The second approach defines the atmospheric CO₂ fugacity as an equilibrium phase with exchange of CO₂ from and to the atmosphere to maintain the equilibrium during the complete simulation. The third approach removes CO₂ from the aqueous-solid system only to prevent supersaturation of pure CO₂. This simulates the evolution of a pure gas phase not taking any volume or pressure changes into account. A further approach including the explicit simulation of a gas phase was only applied to a subset of systems and allows to investigate the influence of a closed system including changes in pressure at fixed volumetric suspension/gas-ratios of 100, 500 and 1000 ml gas phase per kg of water.

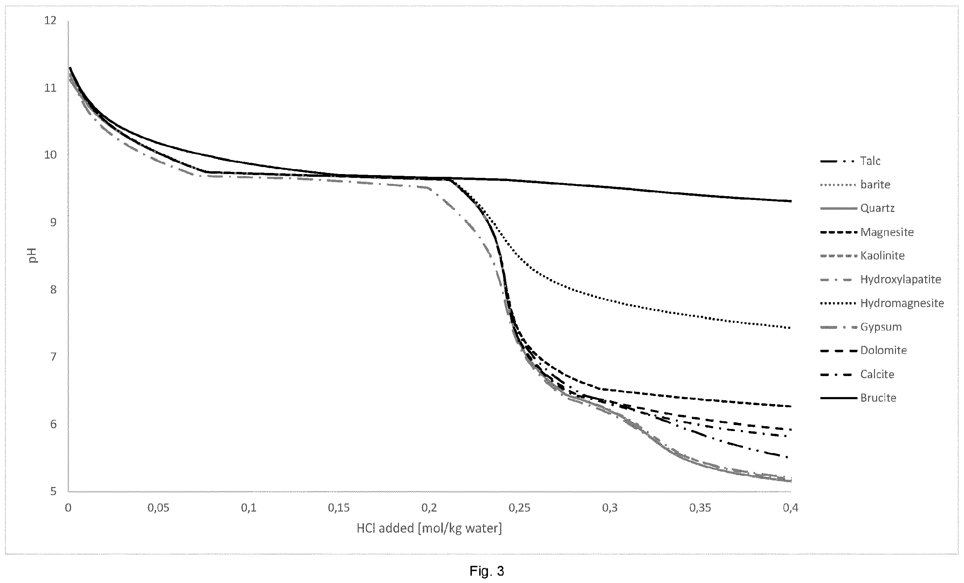

The pH buffer effect was simulated by the stepwise addition of hydrochloric acid to the suspensions defined by the method described above. For every step, the full set of equilibria was calculated and the results were investigated for aqueous composition (including speciation and pH), saturation of solid components included in the database, the concentrations of the supersaturated solids and the composition and properties of gas phase components. The results are shown in Fig. 3 for the aqueous suspensions of the different inorganic particulate materials. Similar results are obtained for the suspensions of different inorganic particulate materials when using lactic-, acetic- and citric acid.

## Claims

1. Buffer composition comprising a first and a second buffer component, wherein
a) the first buffer component is at least one water soluble or water dispersable source of magnesium ions and/or zinc ions, and
b) the second buffer component is at least one alkali carbonate and/or at least one alkali bicarbonate,
wherein the molar ratio of the first buffer component to the second buffer component [Mg and/or Zn ions/ carbonate and/or bicarbonate ions] is from 10 000:1 to 1:10 000.

2. The buffer composition according to claim 1, wherein the at least one alkali carbonate is selected from the group consisting of sodium carbonate, potassium carbonate, lithium carbonate, and mixtures thereof, preferably sodium carbonate and/or lithium carbonate and most preferably lithium carbonate and/or the at least one alkali bicarbonate is selected from the group consisting of sodium bicarbonate, potassium bicarbonate, lithium bicarbonate, and mixtures thereof, preferably sodium bicarbonate and/or lithium bicarbonate and most preferably lithium bicarbonate.

3. The buffer composition according to claim 1 or 2, wherein the at least one water soluble or water dispersable source of magnesium ions is at least one magnesium ion-comprising material, preferably the at least one magnesium ion-comprising material is selected from the group comprising magnesium oxide, magnesium hydroxide, magnesium phosphate, magnesium sulphate, magnesium chloride, magnesium bromide, natural or synthetic hydromagnesite, natural or synthetic brucite, and mixtures thereof, preferably magnesium oxide and/or magnesium hydroxide, and/or the at least one water soluble or water dispersable source of zinc ions is at least one zinc ion-comprising material, preferably the at least one zinc ion-comprising material salt is selected from the group comprising zinc carbonate, zinc oxide, zinc hydroxide, zinc phosphate, zinc and mixtures thereof, preferably zinc oxide.

4. The buffer composition according to any one of claims 1 to 3, wherein the molar ratio of the first buffer component to the second buffer component [Mg and/or Zn ions/ carbonate and/or bicarbonate ions] is from 1 000:1 to 1:1 000, preferably from 100:1 to 1:100, more preferably from 20:1 to 1:20, even more preferably from 10:1 to 1:10 and most preferably from 2:1 to 1:2, e.g. about 1:1.

5. The buffer composition according to any one of claims 1 to 4, wherein the buffer composition is capable of maintaining the pH of an article, preferably an aqueous preparation or a solid article, equal to or below 12, preferably from 4 to 12, more preferably from 6 to 11.5 and most preferably from 8.5 to 10.5.

6. The buffer composition according to any one of claims 1 to 5, wherein the buffer composition is in form of a powder, granules, pellets or tablets.

7. The buffer composition according to any one of claims 1 to 5, wherein the buffer composition comprises a solvent, preferably an organic solvent and/or water.

8. The buffer composition according to any one of claims 1 to 5 or 7, wherein the buffer composition is in form of a solution, dispersion, or suspension, preferably an aqueous solution, dispersion, or suspension.

9. The buffer composition according to any one of claims 1 to 8, wherein the buffer composition is free of buffer components comprising ammonia, methylamine, dimethylamine, trimethylamine, diethylamine, propylamine, butylamine, hexylamine, amino-2-methylpropanol (AMP), monoethanolamine (MEA), monoisopropanolamine (MIPA), triethylenetetramine (TETA), calcium hydroxide and mixtures thereof, preferably amine-based buffer components, monoalcohol-based buffer components, primary alkanol amine-based buffer components, hydroxide-based buffer components and mixtures thereof.

10. Article being an aqueous preparation, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation, comprising the buffer composition according to any one of claims 1 to 9, and/or a solid article, preferably a coating, paint film, lacquer or coating, paper coating, paper, paperboard, adhesive, sealant, pigment, fiber, plaster, plaster-spray, plasterboard, binder, thickener and/or concrete, comprising the buffer composition according to any one of claims 1 to 6 and 9.

11. The article according to claim 10, wherein the article further comprises
(i) at least one inorganic particulate material, preferably the at least one inorganic particulate material is selected from the group comprising natural ground calcium carbonate, natural and/or synthetic precipitated calcium carbonate, surface-reacted calcium carbonate, dolomite, calcium sulphate, kaolin, clay, barite, talcum, quartz, mica, gypsum, aluminium hydroxide, aluminium silicate, titanium dioxide, magnesite, hydromagnesite, hydroxylapatite, perlite, sepiolite, brucite and mixtures thereof, and most preferably the at least one inorganic particulate material comprises natural ground calcium carbonate and/or synthetic precipitated calcium carbonate, and/or
(ii) at least one organic material, preferably the at least one organic material is selected from the group comprising carbohydrates such as starch, sugar, cellulose, modified cellulose and cellulose based pulp, glycerol, hydrocarbons, water-soluble polymers such as polyacrylates, and mixtures thereof.

12. The article according to claim 10 or 11, wherein the article is an aqueous preparation having
(i) a pH value of equal to or below 12, preferably from 4 to 12, more preferably from 6 to 11.5 and most preferably from 8.5 to 10.5, and/or
(ii) a solids content of up to 85.0 wt.-%, preferably from 10.0 to 82.0 wt.-%, and more preferably from 20.0 to 80.0 wt.-%, based on the total weight of the aqueous preparation.

13. The article according to any one of claims 10 to 12, wherein the article is an aqueous preparation and the first buffer component is present in a supersaturated state in the aqueous preparation and/or the second buffer component is present in a supersaturated state in the aqueous preparation.

14. The article according to any one of claims 10 to 13, wherein the first and the second buffer components are present in the article in a total amount of at least 100 ppm, e.g. from 100 to 27 000 ppm, preferably at least 250 ppm, e.g. from 250 to 25 000 ppm, more preferably at least 500 ppm, e.g. from 500 to 20 000 ppm, still more preferably at least 600 ppm, e.g. from 600 to 15 000 ppm, even more preferably at least 750 ppm, e.g. from 750 to 10 000 ppm, and most preferably from 750 to 5 000 ppm, calculated relative to the total weight of the article.

15. Process for buffering an aqueous preparation, said process comprising the steps of
a) providing an aqueous preparation, preferably a paper making formulation, a paper coating formulation, fibre formulation, food formulation, pharmaceutical formulation, cosmetic formulation, plastic formulation, adhesive formulation, metal working fluid, cooling fluid, primer coat, levelling compound, pigment formulation, titanium dioxide slurry, concrete additives formulation, binder formulation, thickener formulation, plaster, coating, lacquer and/or a paint formulation,
b) providing a buffer composition as defined in any one of claims 1 to 9, and
c) contacting and mixing the aqueous preparation of step a) one or more times with the buffer composition of step b) for obtaining the buffered aqueous preparation.

16. Process for buffering a solid article, said process comprising the steps of
a) providing a solid article, preferably a coating, paint film, lacquer or coating, paper coating, paper, paperboard, adhesive, sealant, pigment, fiber, plaster, plaster-spray, plasterboard, binder, thickener and/or concrete comprising the buffer composition according to any one of claims 1 to 6 and 9, and
b) moisten the surface of the solid article of step a) for obtaining the buffered solid article.

17. Use of a buffer composition as defined in any one of claims 1 to 9 for maintaining the pH of an article, preferably an aqueous preparation or a solid article, equal to or below 12, preferably from 4 to 12, more preferably from 6 to 11.5 and most preferably from 8.5 to 10.5.
